# EUROPEAN PATENT SPECIFICATION

(11) **EP 2 611 823 B1**
(45) Date of publication and mention of the grant of the patent: **16.05.2018**
(21) Application number: 11820973.3
(22) Date of filing: 02.09.2011
(51) Int. Cl.: C07K 14/205, A61K 39/106, A61K 39/116, A61K 39/385, A61K 39/39, A61P 31/04, A61P 37/04, C07K 19/00, C07K 7/06, C07K 7/08, C07K 9/00, C12N 15/00, C12N 15/31, C12N 15/62, G01N 33/564, A61K 39/02, C07K 16/00, C07K 16/28, C12P 21/00, A61K 39/00

(54) **PEPTIDE CONTAINING MULTIPLE N-LINKED GLYCOSYLATION SEQUONS**
PEPTID MIT MEHREREN N-GEKOPPELTEN GLYCOSYLIERUNGS-SEQUONEN
PEPTIDES CONTENANT DE MULTIPLES SÉQUENCES ACCEPTRICES DE GLYCOSYLATION LIÉES À N

(30) Priority: 03.09.2010 US 379896 P
(43) Date of publication of application: 10.07.2013
(73) Proprietor: The Governors of the University of Alberta, Edmonton, Alberta T5J 4P6 (CA)
(72) Inventor: SZYMANSKI, Christine, Edmonton Alberta T6G 2E9 (CA); NOTHAFT, Harald, Edmonton Alberta T6G 2E9 (CA)
(74) Representative: V.O.
(86) International application number: PCT/CA2011/050535
(87) International publication number: WO 2012/027850

(56) References cited:
- WO-A2-2006/119987
- WO-A2-2009/104074
- CA-A1- 2 607 595
- CA-A1- 2 711 307
- DATABASE UniProt [Online] 19 September 2006 (2006-09-19), "SubName: Full=Putative uncharacterized protein;", XP002715424, retrieved from EBI accession no. UNIPROT:Q0P8I1 Database accession no. Q0P8I1 & PARKHILL J ET AL: "THE GENOME SEQUENCE OF THE FOOD-BORNE PATHOGEN CAMPYLOBACTER JEJUNIREVEALS HYPERVARIABLE SEQUENCES", NATURE: INTERNATIONAL WEEKLY JOURNAL OF SCIENCE (AND SUPPLEMENTARY INFORMATION), NATURE PUBLISHING GROUP, UNITED KINGDOM, vol. 403, 10 February 2000 (2000-02-10), pages 665-668, XP000906767, ISSN: 0028-0836, DOI: 10.1038/35001088 & DATABASE NUCLEOTIDE & DATABASE GENBANK 14 August 2006 (2006-08-14),
- KOWARIK M ET AL: "Definition of the bacterial N-glycosylation site consensus sequence", EMBO JOURNAL, OXFORD UNIVERSITY PRESS, SURREY, GB, vol. 25, no. 9, 13 April 2006 (2006-04-13) , pages 1957-1966, XP002408605, ISSN: 0261-4189, DOI: 10.1038/SJ.EMBOJ.7601087
- IHSSEN JULIAN ET AL: "Production of glycoprotein vaccines in Escherichia coli", MICROBIAL CELL FACTORIES, BIOMED CENTRAL, LONDON, NL, vol. 9, no. 1, 11 August 2010 (2010-08-11) , page 61, XP021077209, ISSN: 1475-2859, DOI: 10.1186/1475-2859-9-61
- Nichollas E Scott ET AL: "Simultaneous glycan-peptide characterization using hydrophilic interaction 1 chromatography and parallel fragmentation by CID, HCD and ETD-MS applied to the N- 2 linked glycoproteome of Campylobacter jejuni 3 4", , 1 April 2010 (2010-04-01), pages 1-48, XP055085375, Retrieved from the Internet: URL:http://www.mcponline.org/content/early /2010/04/01/mcp.M000031-MCP201.full.pdf [retrieved on 2013-10-25]
- DATABASE GENBANK 14 August 2006 XP008163705 Database accession no. CAL35542
- PARKHILL J. ET AL.: 'The genome sequence of the food-borne pathogen Campylobacter jejuni reveals hypervariable sequences' NATURE vol. 403, 10 February 2000, pages 665 - 668, XP000906767 & DATABASE NUCLEOTIDE [Online] Retrieved from NCBI Database accession no. AL111168
- KOWARIK, M. ET AL.: 'Definition of the bacterial N-glycosylation site consensus sequence' EMBO J. vol. 25, no. 9, 13 April 2006, pages 1957 - 1966, XP002408605

## Description

### CROSS-REFERENCE TO RELATED APPLICATION

This application claims priority to U.S. provisional patent application No. 61/379,896, filed September 3, 2010.

### FIELD OF THE INVENTION

The invention relates to campylobacter-derived peptides which contain multiple sequential sequons for *N*-linked glycosylation.

### BACKGROUND

Asparagine (*N*-linked) protein glycosylation was once believed to exist in only eukaryotes and archaea until its discovery in the ε-Proteobactenum *Campylobacter jejuni* (Szymanski CM et al., 1999: Evidence for a system of general protein glycosylation in Campylobacter jejuni. Mol Micro, 32(5): 1022-1030; Wacker M et al., 2002: N-linked glycosylation in Campylobacter jejuni and its functional transfer into E. coli. Science, 298(5599):1790-1793; Young NM et al., 2002: Structure of the N-linked glycan present on multiple glycoproteins in the Gram-negative bacterium, Campylobacter jejuni. J Biol Chem, 277(45):42530-42539). In eukaryotes, *N*-linked glycosylation is an essential process and is catalyzed by an oligosaccharyltransferase (OST) complex consisting of 9 proteins with STT3 being the catalytic subunit (for a review see: Yan A & Lennarz WJ., 2005: Unraveling the mechanism of protein N-linked glycosylation. J Biol Chem, 280(5):3121-3124). In *C. jejuni, N*-linked glycosylation occurs in the periplasm and PglB, the STT3 orthologous protein, is the key enzyme responsible for the transfer of oligosaccharides to acceptor peptides and proteins. The presence of a consensus sequence (an *N*-linked glycosylation sequon) within the secreted acceptor peptides is required for recognition by PglB. This sequon consists of D/E-X₁-N-X₂-S/T, where X₁ and X₂ can be any amino acid except proline (Kowarik M et al., 2006: Definition of the bacterial N-glycosylation site consensus sequence. EMBO J, 25(9):1957-1966).

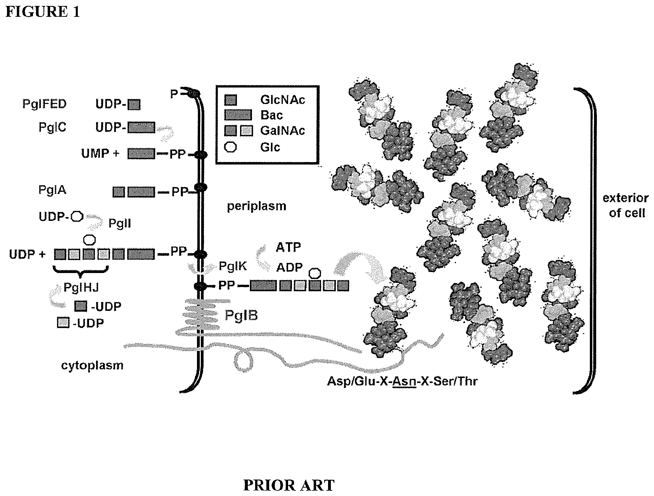

The current model for assembly of the *C. jejuni N*-linked glycan is shown in Figure 1 (Szymanski CM et al., 2003: Campylobacter - a tale of two protein glycosylation systems. Trends Microbiol, 5(11): 233-238). The bactoprenyl-pyrophosphate-linked heptasaccharide is assembled in the cytosol by the addition of nucleotide-activated sugars (Szymanski CM et al., 2003: Campylobacter - a tale of two protein glycosylation systems. Trends Microbiol, 5(11): 233-238; Szymanski CM et al., 2003: Detection of conserved N-linked glycans and phase-variable lipooligosaccharides and capsules from campylobacter cells by mass spectrometry and high resolution magic angle spinning NMR spectroscopy. J Biol Chem, 278(27): 24509-24520). The complete heptasaccharide is translocated across the inner membrane to the periplasm by the ATP-Binding Cassette (ABC) transporter PglK (Alaimo C et al., 2006: Two distinct but interchangeable mechanisms for flipping of lipid-linked oligosaccharides. EMBO J, 25(5):967-976). The oligosaccharide is then transferred to an *N*-linked glycosylation sequon by PglB or released into the periplasmic space as free oligosaccharide (fOS) (Nothaft et al., 2009: Study of free oligosaccharides derived from the bacterial N-glycosylation pathway. PNAS, 106(35): 15019-15024). (Young NM et al., 2002: Structure of the N-linked glycan present on multiple glycoproteins in the Gram-negative bacterium, Campylobacter jejuni. J Biol Chem, 277(45): 42530-42539; Wacker M et al., 2002: N-linked glycosylation in Campylobacter jejuni and its functional transfer into E. coli. Science, 298(5599):1790-1793).

It has been demonstrated that the *N*-linked protein glycosylation machinery of *C. jejuni* can be functionally transferred into *Escherichia coli* (Wacker M et al., 2002: N-linked glycosylation in Campylobacter jejuni and its functional transfer into E. coli. Science, 298(5599):1790-1793). This has led to a surge of activity to exploit this pathway for glycoprotein engineering and has opened up the possibility of engineering glycan structures for various purposes, which include but are not limited to, furthering research of *N*-linked glycosylated proteins and *N*-linked glycosylation, and therapeutic applications including the production of therapeutically useful antibodies and vaccines.

However, *N*-linked glycoprotein engineering using the *C. jejuni* glycosylation machinery in *E. coli* has several disadvantages and limitations. One of the most important limitations to recombinant glycoprotein production is the number of *N-*linked glycosylation sequons present within a natural glycoprotein. As one of skill in the art will appreciate, currently available molecular biology techniques allow for the insertion of additional *N*-linked glycosylation sequons in naturally-glycosylated proteins and even the insertion of *N*-linked glycosylation sequons in proteins that are not normally glycosylated. However, insertion of these *N*-linked glycosylation sites follows a mostly trial-and-error approach. Without significant knowledge of a protein's three-dimensional structure, some *N*-linked glycosylation sites may be inserted in regions of the protein that will not be accessible to the protein glycosylation machinery (Kowarik M et al., 2006: N-linked glycosylation of folded proteins by the bacterial oligosaccharyltransferase. Science, 314 (5802): 1148-1150; Kowarik M et al., 2006: Definition of the bacterial N-linked glycosylation site consensus sequence. EMBO J, 25(9):1957-1966). Moreover, lack of information on the three-dimensional structure of a protein may prevent the insertion of sequential *N-*linked glycosylation sequons. The *N*-linked glycosylation sequon identified by Kowarik M *et al.* (2006) was identified as forming a segment within a protein, but wherein only a single such sequon was identified within a protein, or the sequons were widely spaced within the protein. Without structural information of proteins, it is very difficult to introduce multiple *N*-linked glycosylation sequons or sequential sequons in a protein and ensure efficient glycosylation in a recombinant host.

Furthermore, the engineering of *N*-linked glycosylation sequons in a protein or peptide is a time-consuming and often costly process. This can be particularly problematic for the development of therapeutic antibodies and/or vaccines.

Consequently, the need has arisen for peptides and proteins that contain multiple *N*-linked glycosylation sequons, that can be efficiently and recombinantly glycosylated, while avoiding some of the problems listed above.
Uniprot:Q0P8I1 (Database UniProt 19 September 2006), also referred to as Genbank Nucleotide: Cj1433c, and Parkhill J. et al., 2000: The genome sequence of the food-borne pathogen Campylobacter jejuni reveals hypervariable sequences. Nature, 403:665-668, disclose a hypothetical protein from *Campylobacter jejuni* comprising nine contiguous units of the amino acid sequence as set forth in SEQ ID NO: 1. Ihssen J. et al., 2010: Production of glycoprotein vaccines in Escherichia coli. Microbial Cell Factories, 9:61 discloses the *in vivo* biosynthesis of two conjugate vaccine candidates against *Shigella dysenteriae* type 1. Two different periplasmic carrier proteins, AcrA from *C. jejuni* and a toxoid form of *Pseudomonas aeruginosa* exotoxin were glycosylated with *Shigella* O antigens in *E. coli.*
WO 2009/104074 discloses a bioconjugate vaccine comprising a protein carrier comprising an inserted consensus sequence D/E-X-N-Z-S/T, where X and Z can be any amino acid except proline; at least one antigenic polysaccharide from at least one bacterium, linked to the protein carrier, wherein the at least one antigenic polysaccharide is at least one bacterial O-antigen from one or more strains of *Shigella, E. coli* or *Pseudomonas aeruginosa;* and, optionally, an adjuvant. A method of producing an O1-bioconjugate in a bioreactor is also described.
WO 2006/119987 similarly discloses recombinant N-glycosylated proteins comprising one or more introduced consensus sequence(s) as described above and methods for their production. The consensus sequence is efficiently N-glycosylated by the oligosaccharyl transferase (OST, OTase) from *Campylobacter* spp., preferably *C. jejuni.*

This background information is provided for the purpose of making known information believed by the applicant to be of possible relevance to the present invention. No admission is necessarily intended, nor should be construed, that any of the preceding information constitutes prior art against the present invention.

### SUMMARY

The object of the present application is to provide a peptide containing multiple *N*-linked glycosylation sequons, and methods of preparation and use thereof. The present invention provides: a peptide as defined in claim 1 appended hereto; a chimeric protein as defined in appended claim 3; a method for producing a chimeric protein as defined in appended claim 4; uses of the peptide and/or chimeric protein of the invention as defined in appended claims 10 and 11; and a vaccine, a method for diagnosing a bacterial infection and an immunoassay kit for the same as defined in appended claim 12, 13 and 15, respectively.
In accordance with a broad aspect, there is provided a peptide comprising at least two copies of the amino acid sequence set forth in SEQ ID NO: 1 (this peptide is referred to herein as a "GlycoTag" peptide), wherein the copies of the amino acid sequence set forth in SEQ ID NO: 1 are contiguous or separated by no more than 6 amino acids, wherein the peptide is glycosylated at at least one of the copies of the amino acid sequence set forth in SEQ ID NO: 1. In one embodiment, the GlycoTag peptide comprises nine copies of the amino acid sequence set forth in SEQ ID NO: 1. The GlycoTag peptide can further comprise a signal peptide that facilitates periplasmic secretion.

In accordance with another broad aspect, there is provided a chimeric protein comprising at least one GlycoTag peptide and a second peptide or protein. The GlycoTag peptide comprises at least two copies of the amino acid sequence set forth in SEQ ID NO: 1, which are contiguous or separated by no more than 6 amino acids, wherein the peptide is glycosylated at at least one of the copies of the amino acid sequence set forth in SEQ ID NO: 1. In one embodiment, the second peptide or protein comprises a protein derived from, or native to, a *Campylobacter.* In one embodiment, the second protein is AcrA. In one embodiment, the second protein or peptide is selected from the group consisting of a toxin, a toxoid, an antibody and a periplasmic protein. The toxin or toxoid may be derived from the group selected from *Bordetella pertussis, Clostridium tetani,* and *Corynebacterium diphtheriae.* In one embodiment, the antibody selected as the second protein or peptide targets a dendritic cell.

In accordance with another broad aspect, there is provided a method for producing a chimeric protein comprising multiple glycosylation sites, the method comprising the steps of engineering a chimeric gene comprising a nucleic acid sequence encoding a GlycoTag peptide operably linked, directly or via a linker polynucleotide, to a nucleic acid sequence encoding a second peptide or protein (for example, a protein or peptide that is capable of targeting the chimeric protein to the periplasmic space), and expressing the resulting chimeric gene in a host. The host can be *E. coli.*

In one embodiment, the second peptide or protein comprises a toxin or toxoid, an antibody or a periplasmic protein. In one embodiment, the toxin or toxoid is derived from *Bordetella pertussis, Clostridium tetani,* or *Corynebacterium diphtheriae.* In one embodiment, the second protein comprises an antibody. In that case, the chimeric protein may be for use in an adjuvant-free composition for use in treating a Campylobacter infection. In one embodiment, the antibody targets a dendritic cell. In one embodiment, the second protein or peptide is derived from, or native to, a Campylobacter. In another embodiment, the second protein is AcrA.

In one embodiment, the method for producing a chimeric protein comprises the further step of glycosylating the chimeric peptide or protein with a plurality of *N-*linked glycans. In one embodiment, the *N*-linked glycans are derived from *C*. *jejuni.*

The GlycoTag peptide described herein can be used for many different purposes. In one embodiment, the GlycoTag peptide can be used to display *N-*glycans of eukaryotic, bacterial and/or archaeal origin. In another embodiment, it can be used to display *O*-glycans. In one embodiment, the GlycoTag peptide when glycosylated with specific glycans can be used as a glycan carrier in a vaccine which is for vaccination against zoonotic human pathogens selected from the group consisting of *Campylobacter upsaliensis* in cats and dogs, *Campylobacter coli* in pigs and *Campylobacter jejuni* in chickens. In another embodiment, it can be used as a glycan carrier in a vaccine which is for vaccination against Travelers' Diarrhea caused by *Campylobacter jejuni.* In another embodiment, it can be used as a glycan carrier in a vaccine which is for vaccination of cows and sheep to prevent infertility and abortions caused by *Campylobacter fetus venerealis* and *Campylobacter fetus fetus.* In yet another embodiment, it can be used in a diagnostic method in which it is used to display bacterial glycans and to then determine the presence or absence, or to quantify, anti-sera in a sample obtained from a subject suspected of having a bacterial infection.

In another embodiment, the GlycoTag peptide of the present invention can be used as an adjuvant in vaccine preparation. It can also be used in combination with a second protein or peptide for the preparation of a vaccine or pharmaceutical. The second peptide or protein can comprise a protein derived from or native to a Campylobacter. In one embodiment, the second protein is AcrA. In one embodiment, the second protein or peptide is selected from the group consisting of a toxin, toxoid, an antibody and a periplasmic protein. The toxin or toxoid can be derived from *Bordetella pertussis, Clostridium tetani,* or *Corynebacterium diphtheriae.* In one embodiment, the antibody selected as the second protein targets a dendritic cell.

### BRIEF DESCRIPTION OF THE DRAWINGS

The present invention, both as to its organization and manner of operation, may best be understood by reference to the following description, and the accompanying drawings of various embodiments wherein like numerals are used throughout the several views, and in which:
FIG. 1 is a schematic diagram of the process of *N*-linked glycosylation in *C*. *jejuni* as determined in the prior art.

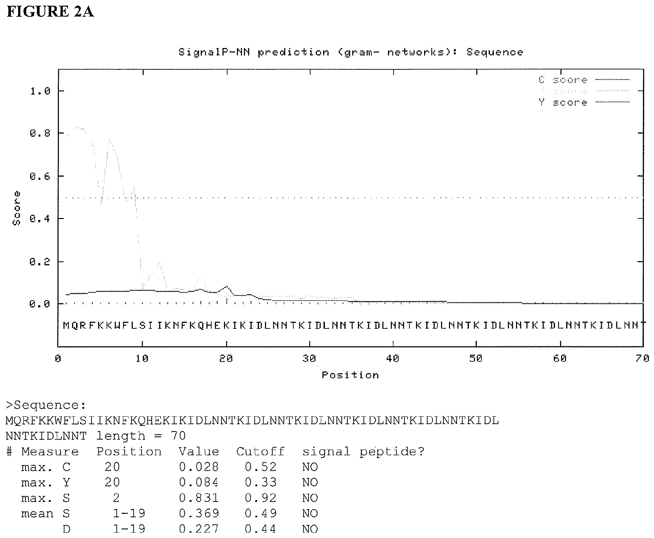

FIG. 2A is the graphical output from a bioinformatic analysis of a segment of the Cj 1433c protein using SignalP-NN. The first 70 amino acids of the 1433c protein were analyzed with the SignalP program (version 3.0) (http://www.cbs.dtu.dk/services/SignalP/) to evaluate the possibility of Cj 1433c being a periplasmic protein. The algorithm clearly shows the absence of a signal peptide sequence necessary for secretion into the periplasmic space of *C. jejuni,* which is a prerequisite for *N*-linked protein glycosylation.

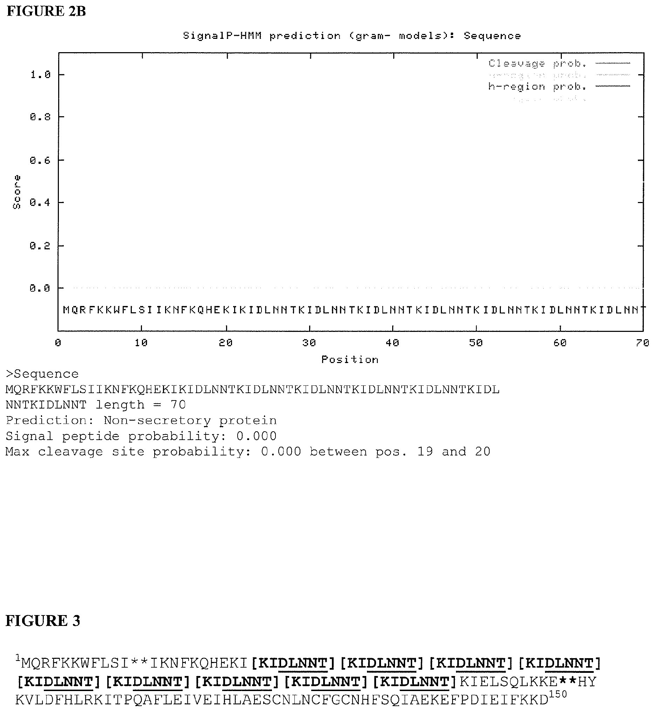

FIG. 2B is the graphical output from a bioinformatic analysis of a segment of the Cj 1433c protein using SignalP-HMM. The first 70 amino acids of the 1433c protein were analyzed with the SignalP program (version 3.0) (http://www.cbs.dtu.dk/services/SignalP/) to evaluate the possibility of Cj 1433c being a periplasmic protein. The algorithm clearly shows the absence of a signal peptide sequence necessary for secretion into the periplasmic space of *C. jejuni,* which is a prerequisite for *N*-linked protein glycosylation.
FIG. 3 shows the sequence of the first 150 amino acids of the *C. jejuni* 1433c protein (SEQ ID NO: 31). The sequence containing the GlycoTag peptide (nine repeats of SEQ ID NO: 1) is located between ** and **. The bacterial *N-*glycosylation sequon (DLNNT) is underlined.

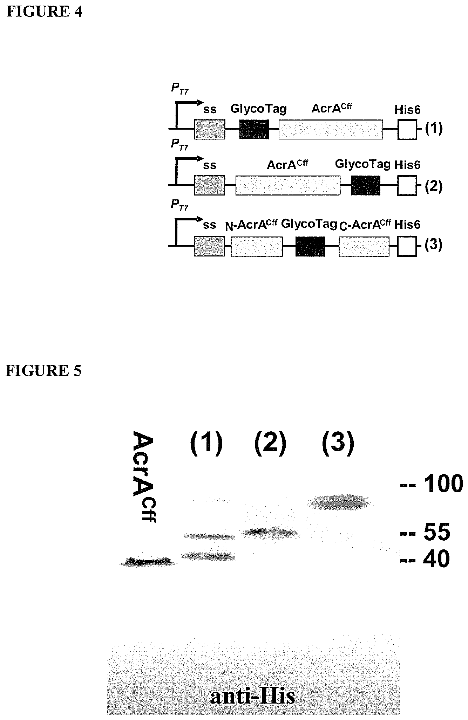

FIG. 4 shows the composition of construct nos. 1, 2 and 3, where the GlycoTag was fused to an AcrA construct. For constructs 1, 2 and 3, AcrA, an *N-*glycoprotein of *Campylobacter fetus fetus* (Cff), was used (AcrA^{Cff}). The GlycoTag peptide was inserted at the N-terminus in construct 1 (to yield GlycoTag-AcrA^{Cff}-His fusion protein), at the C-terminus in construct 2 (to yield AcrA^{Cff}-GlycoTag-His fusion protein), and in between the lipoyl- and the α-hairpin domain of the AcrA protein in construct 3 (to yield N-AcrA^{Cff}-GlycoTag-C-AcrA^{Cff}-His fusion protein). "ss" stands for pectate lyaseB leader sequence, while "His6" stands for a hexa-histidine tag.
FIG. 5 depicts a photograph of an SDS-PAGE gel demonstrating the expression of the first generation of GlycoTag-AcrA^{Cff} fusion proteins produced using the constructs described in FIG. 4. The pectate lyaseB leader sequence (ss) was used to direct the protein to the periplasm. Expression of the fusion proteins in *E. coli* BL21 grown at 28°C was followed by western blotting (anti-His) after 10% SDS-PAGE of cells after induction with 0.1 mM IPTG for 18 h. Only the C-terminal fusion construct (construct 2, lane 2) resulted in a protein with the expected size of 55 kDa.

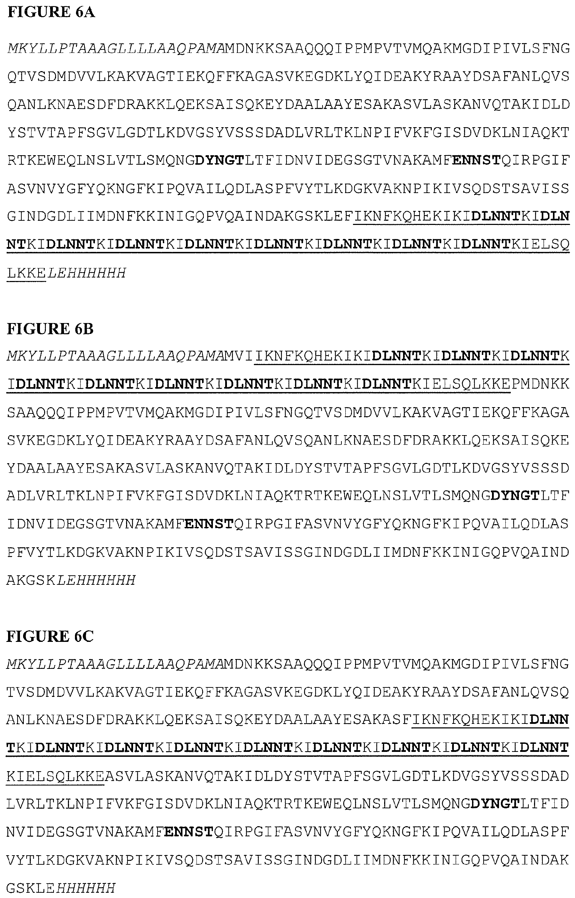

FIG. 6A is the amino acid sequence of the AcrA^{Cff}-GlycoTag-His fusion protein as per construct 1 shown in FIG. 4. The pET22b-derived *pelB* leader (ss) and hexa-histidine amino acid sequences are in italics, the *N*-linked glycosylation sequons are in bold and the GlycoTag peptide is underlined.
FIG. 6B is the amino acid sequence of the GlycoTag-AcrA^{Cff}-His fusion protein as per construct 2 shown in FIG. 4. The pET22b-derived *pelB* leader (ss) and hexa-histidine amino acid sequences are in italics, the *N*-linked glycosylation sequons are in bold and the GlycoTag peptide is underlined.
FIG. 6C is the amino acid sequence of the interdomain N-AcrA^{Cff}-GlycoTag-C-AcrA^{Cff}-His fusion protein as per construct 3 shown in FIG. 4. The pET22b-derived *pelB* leader (ss) and hexa-histidine amino acid sequences are in italics, the *N-*linked glycosylation sequons are in bold and the GlycoTag peptide is underlined.

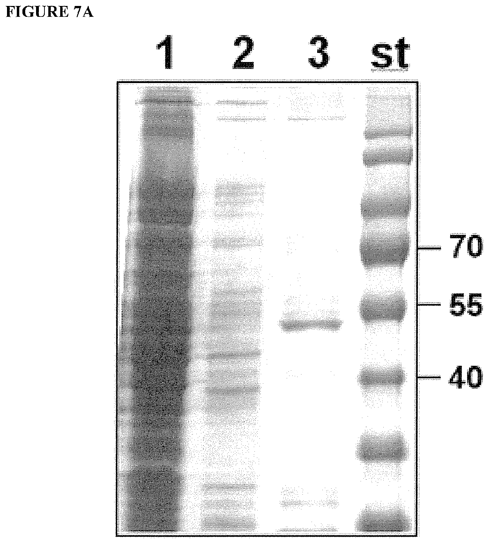

FIG. 7A depicts a photograph of an electrophoretogram showing purification of the soluble, periplasmically-expressed AcrA^{Cff}-GlycoTag-His fusion protein (construct 2) in the presence of the *pgl* machinery of *C. jejuni.* Ni-NTA affinity chromatography was used for purification. Lane 1 is a sample of crude cell lysate; lane 2 is a sample of column flow-through; and lane 3 is a sample of the eluate obtained with 250 mM imidazole.

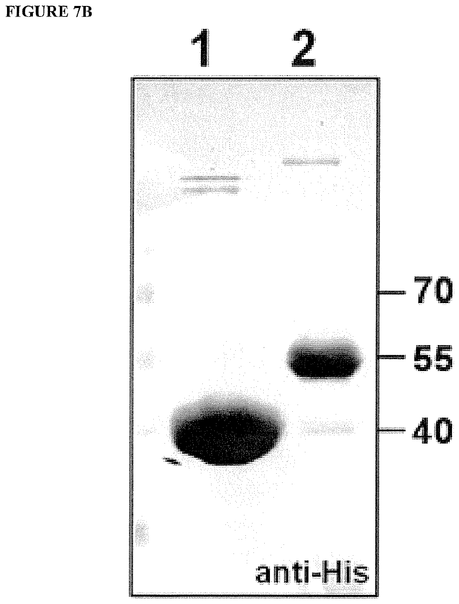

FIG. 7B depicts a photograph of an immunoblot of His-tagged proteins produced by western blotting with anti-His antibodies. Lane 1 is a sample of the AcrA^{Cff} protein and lane 2 is a sample of the AcrA^{Cff}-GlycoTag-His fusion protein.

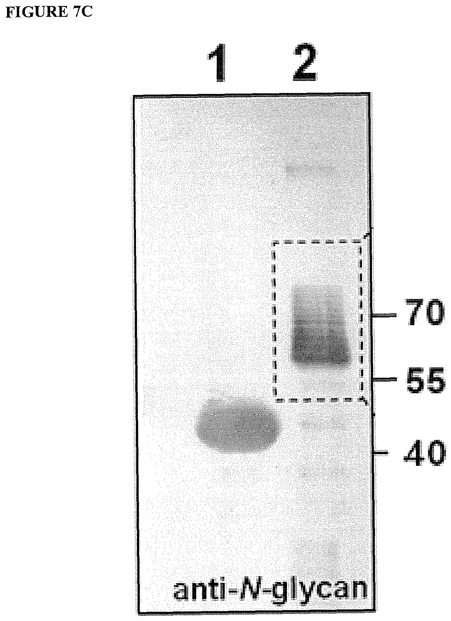

FIG. 7C depicts a photograph of an immunoblot of His-tagged proteins produced by western blotting with *C. jejuni N*-glycan-specific antiserum. Lane 1 is a sample of the AcrA^{Cff} protein and lane 2 is a sample of the AcrA^{Cff}-GlycoTag-His fusion protein.

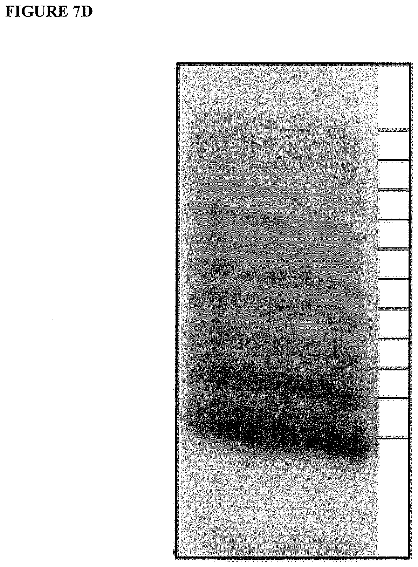

FIG. 7D is an expanded view of lane 2 of FIG. 7C, showing the *N-*glycosylated fusion protein with up to 11 heptasaccharides.

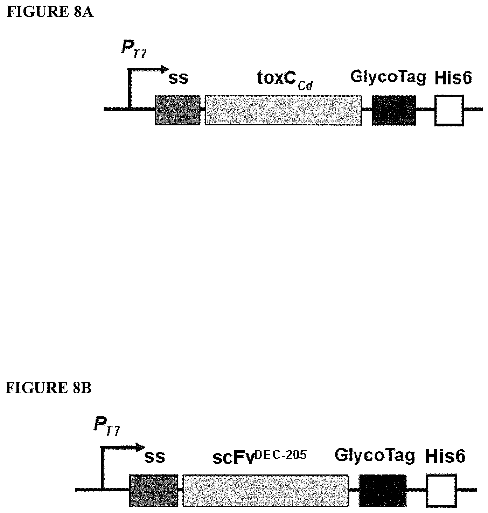

FIG. 8A is a schematic diagram of a construct used to produce a toxoid-GlycoTag fusion protein in the heterologous *E. coli* protein glycosylation system (toxC*_{cd}*-GlycoTag fusion protein). The GlycoTag was fused to the C-terminus of the N-terminal portion (nt 79 to nt 654) of the *toxC* gene of *Corynebacterium diphtheriae* NCTC 13129. "ss" stands for pectate lyaseB leader sequence, while "His6" stands for a hexa-histidine tag.
FIG. 8B is a schematic diagram of a construct used to produce a dendritic cell targeting single chain antibody-GlycoTag fusion protein (scFv-GlycoTag fusion protein) in the heterologous *E. coli* protein glycosylation system. The GlycoTag was fused to the C-terminus of a scFv that recognizes the DEC-205 receptor on mouse dendritic cells, "ss" stands for pectate lyaseB leader sequence, while "His6" stands for a hexa-histidine tag.

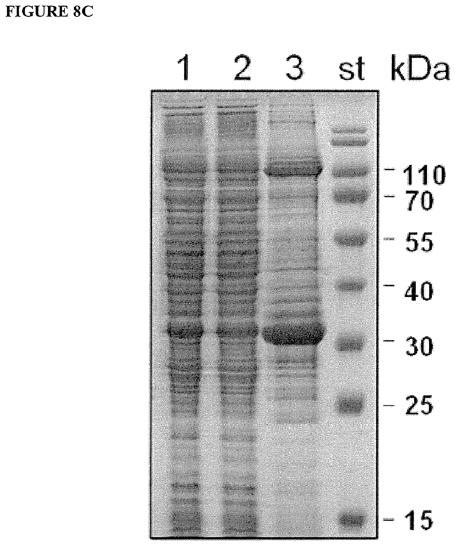

FIG. 8C depicts a photograph of an electrophoretogram of a 10% SDS-PAGE gel showing expression of the toxC*_{Cd}*-GlycoTag fusion protein in the heterologous *E*. *coli* protein glycosylation system. The fusion protein was purified using Ni-NTA affinity chromatography. Lane 1 is a sample of whole cell lysate of cells after induction of protein expression with 0.1 mM IPTG for 18 h; lane 2 is a sample of flowthrough from the Ni-NTA affinity chromatography; and lane 3 is a sample of protein eluted with 250 mM imidazole.

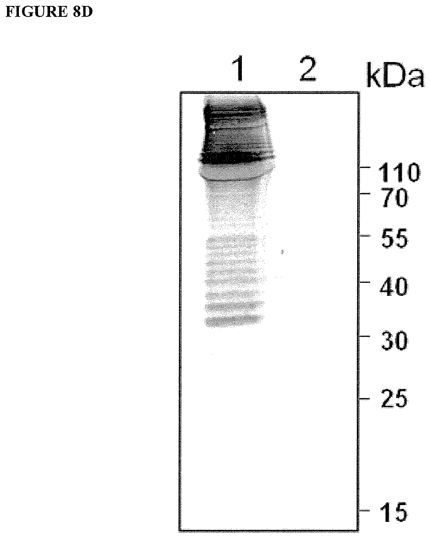

FIG. 8D depicts a photograph of an electrophoretogram showing immunodetection of the glycosylation of the toxC*_{Cd}*-GlycoTag fusion protein with *Campylobacter jejuni* N-glycan specific hR6 antiserum in *E. coli* (*pgl*+)*,* lane 1, or *E*. *coli* (*pgl*-), lane 2.

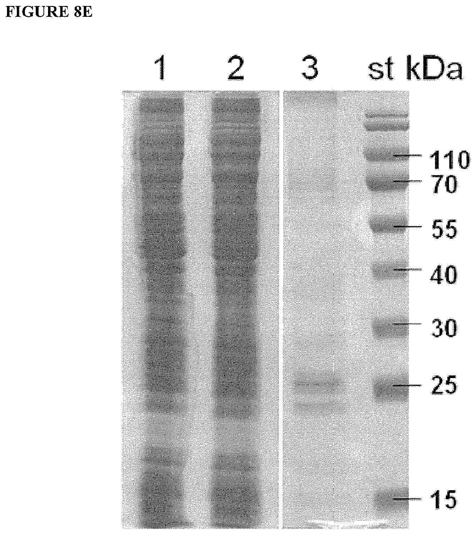

FIG. 8E depicts a photograph of an electrophoretogram of a 10% SDS-PAGE gel showing expression of the scFv-GlycoTag fusion protein in the heterologous *E*. *coli* protein glycosylation system. The fusion protein was purified using Ni-NTA affinity chromatography. Lane 1 is a sample of whole cell lysate of cells after induction of protein expression with 0.1 mM IPTG for 18 h; lane 2 is a sample of flowthrough from the Ni-NTA affinity chromatography; and lane 3 is a sample of protein eluted with 250 mM imidazole.

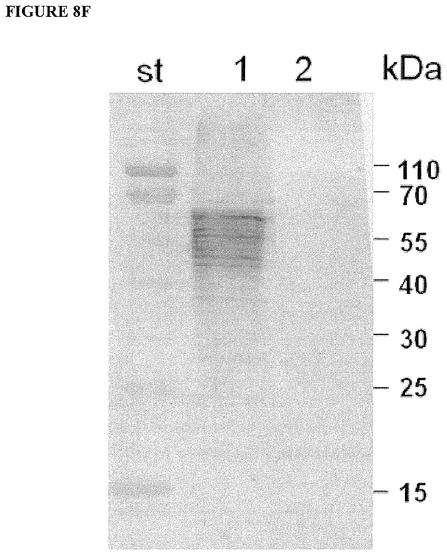

FIG. 8F depicts a photograph of an electrophoretogram showing immunodetection of the glycosylation of the scFv-GlycoTag fusion protein with hR6 antiserum in *E. coli* (*pgl*+), lane 1, or *E. coli* (*pgl*-), lane 2.

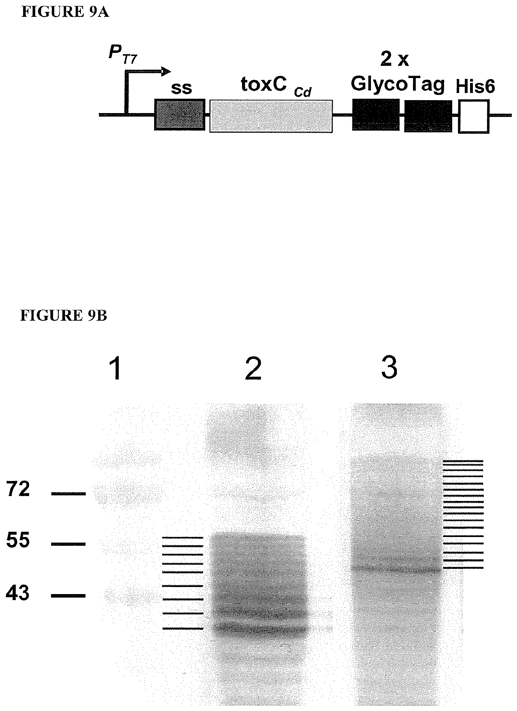

FIG. 9A is a schematic diagram of a construct used to produce a toxoid-(GlycoTag)₂ fusion protein in the heterologous *E. coli* protein glycosylation system (toxC*_{cd}*-(GlycoTag)₂ fusion protein). Two sequential copies of the GlycoTag peptide were fused to the C-terminus of the N-terminal portion (nt 79 to nt 654) of the *toxC* gene of *Corynebacterium diphtheriae* NCTC 1312. "ss" stands for pectate lyaseB leader sequence, while "His6" stands for a hexa-histidine tag.
FIG. 9B illustrates detection of the toxC*_{Cd}*-GlycoTag and toxC*_{Cd}*-(GlycoTag)₂ fusion proteins expressed and glycosylated with the *C. jejuni* heptasaccharide. A western blot with hR6 antiserum after 12% SDS-PAGE of Ni-NTA affinity-purified (as described above in FIG. 8) toxC*_{Cd}*-GlycoTag protein (lane 2) and toxC*_{Cd}*-(GlycoTag)₂ (lane 3) fusion proteins. Protein expression in *E. coli* BL21 in the presence of the *C. jejuni* protein glycosylation operon on plasmid pACYC was induced with 0.1 mM IPTG for 18 h. For clarity, individual glycoprotein-specific signals are marked by a line on the side of the corresponding signal. The sizes of molecular weight markers in kDa (lane 1) are indicated on the left.

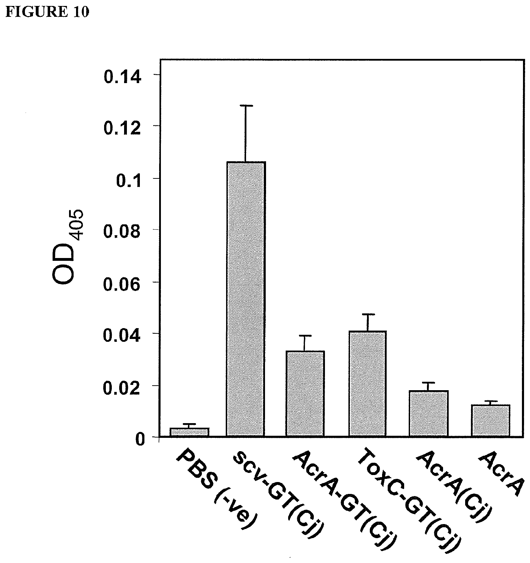

FIG. 10 is a bar chart summarizing the *N*-glycan-specific antibody response. ELISA Plates (Nunc 96-well Maxisorb) were coated overnight with monovalent LipidA-*N*-glycan conjugate (prepared according to Manoharan R et al., 1990: UV resonance Raman spectra of bacteria, bacterial spores, protoplasts, and calcium dipicolinate. J. Microbiol. Methods 11(1): 1-15) from an *E. coli* O-antigen polymerase mutant strain that expresses the protein glycosylation gene cluster of *C. jejuni* (Wacker M et al., 2002: N-linked glycosylation in Campylobacter jejuni and its functional transfer into E. coli. Science, 298(5599):1790-1793)). Plates were washed 3-times with PBST (0.1% Tween 20 in PBS, pH 7.3) followed by a blocking step with 2% skim milk for 3 h at RT. Plates were washed once with PBST and the 1:50 diluted (in PBST) mouse sera were added and incubated for 2 h at RT. Plates were then washed with PBST and incubated with alkaline-phosphatase labeled anti-mouse antibody (Santa Cruz, 1:1000 dilution in PBST). After 1 h at room temperature, the plates were washed 3-times with PBST, PNPP was added to each well, and the OD₄₀₅ nm and the OD₅₇₀ nm (background) was taken after 30 min using a microplate reader. Each bar represents the background subtracted values for the indicated antigen group; standard deviations that represent 2 experimental and 2 technical replicates are indicated by error bars.

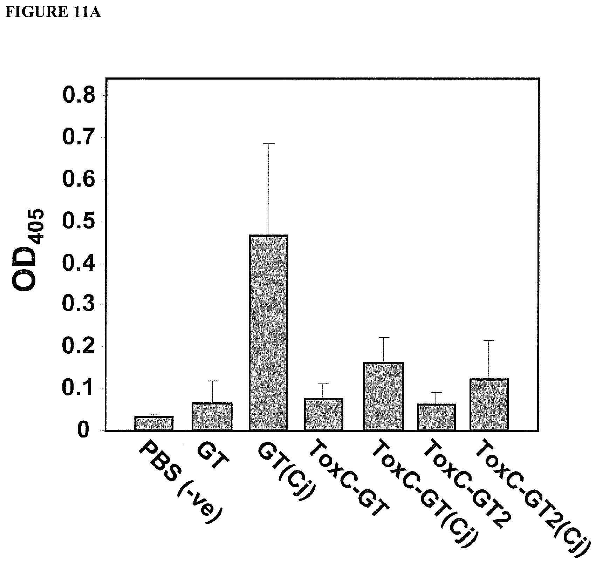

FIG. 11A is a bar chart summarizing the *N*-glycan-specific antibody response from day 21. ELISA plates were coated as described (FIG 10). Analysis of the *N-*glycan-specific immune response using diluted mouse sera (1:100 in PBST) was done as described (in FIG 10).

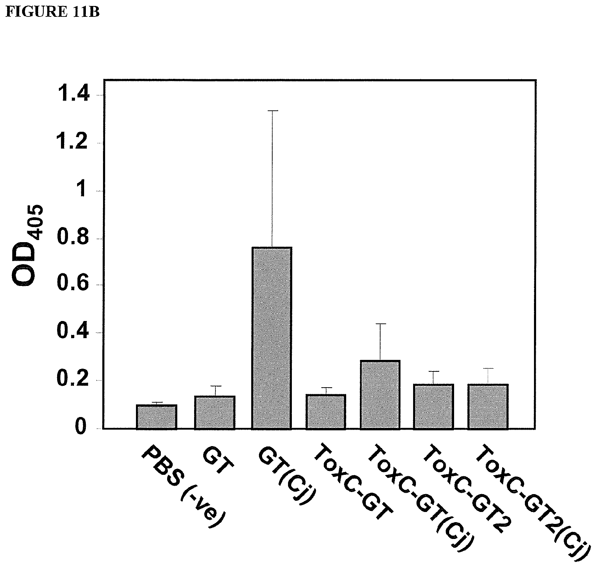

FIG. 11B is a bar chart summarizing the *N*-glycan-specific antibody response from day 35. ELISA plates were coated as described (FIG 10). Analysis of the *N-*glycan-specific immune response using diluted mouse sera (1:100 in PBST) was done as described (in FIG 10).

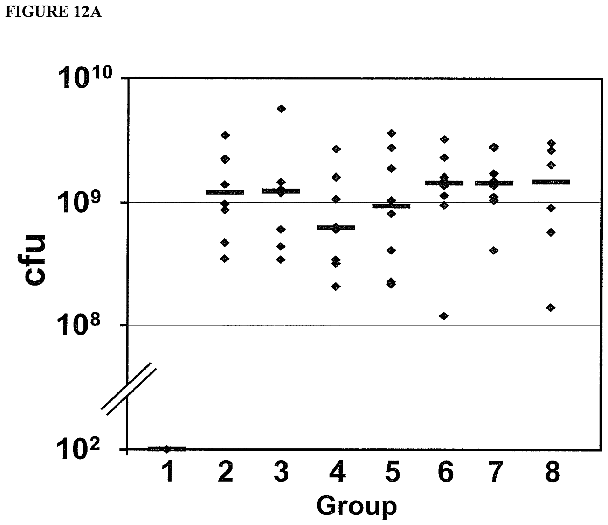

FIG. 12A represents the colony counts (cfu) obtained after colonization of 1-day-old chickens by *Campylobacter jejuni* in the groups as outlined in table 3. The bar represents the median level of colonization for each group.

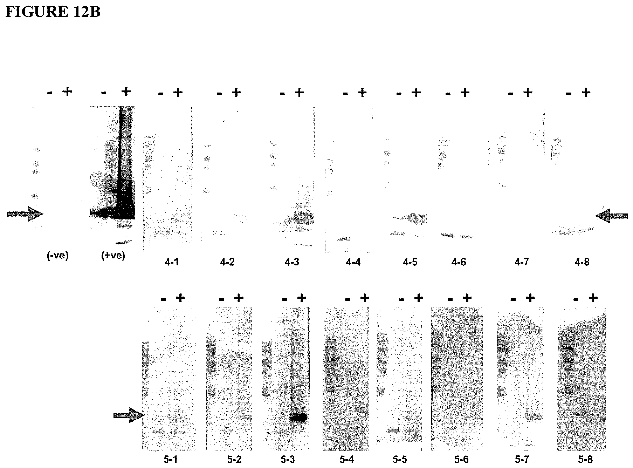

FIG. 12B illustrates the IgG (IgY) humoral response in group 4 and group 5 chicken sera after immunoblotting against Proteinase K digested *E. coli* extracts producing monovalent Lipid A-linked *N*-glycan of *C. jejuni* (+) or LipidA core (-) that were separated on 16% DOC gels and probed with sera from different chicken (final bleed, [group-chicken number as indicated]). The molecular weight of the *N-*glycan-specific signals when compared to the positive control (positive [+ve] control) probed with *N*-glycan-specific antiserum, hR6, are indicated by an arrow. No *N-*glycan-specific signal was obtained with pooled sera obtained from the final bleed of group 1 chickens (negative [-ve] control).

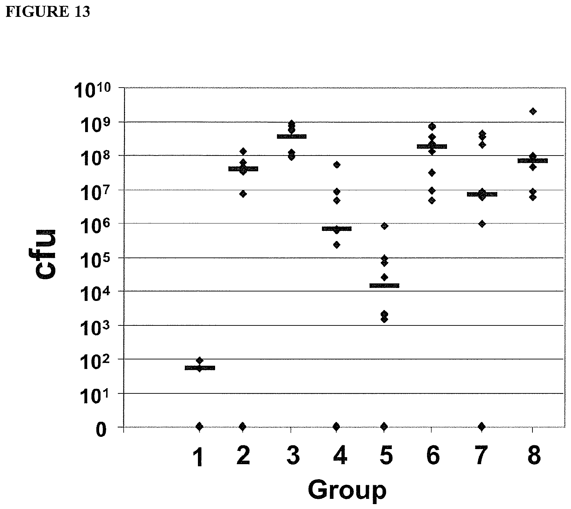

FIG. 13 represents the colony counts (cfu) obtained after colonization of 1-day-old chickens by *Campylobacter jejuni* in the groups as outlined in table 4. The bar represents the median level of colonization for each group.

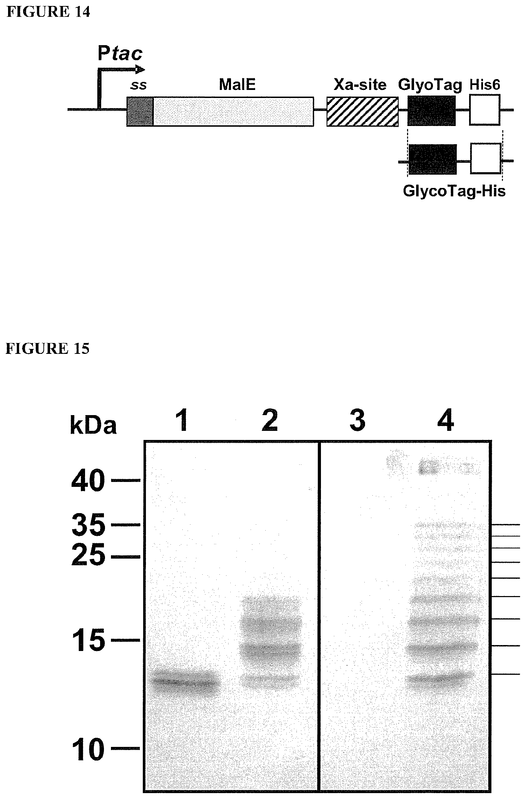

FIG. 14 is a schematic diagram of a construct used to produce a Maltose-binding-protein-factorXa-GlycoTag-hexa-histidine fusion protein in the heterologous *E. coli* protein glycosylation system (MalE-GT-His). The GlycoTag is fused to the C-terminus of MalE that can be purified using amylose-agarose affinity chromatography. "Ptac" stands for IPTG-inducible promoter, "MalE" stands for Maltose-binding-protein, "Xa-site" stands for factor Xa protease recognition sequence "ss" stands for pectate lyaseB leader sequence, while "His6" stands for a hexa-histidine tag.
FIG. 15 illustrates detection of the GlycoTag peptide and detection of the GlycoTag peptide glycosylated with the *C. jejuni* heptasaccharide. Western blots with His-Tag-specific and *Campylobacter jejuni N*-glycan specific hR6 antiserum after 15% SDS-PAGE of Ni-NTA affinity-purified (as described above in FIG. 8) GlycoTag after digestion of amylose-agarose-affinity purified MalE-GlycoTag fusion protein with factor Xa protease (lane 1, His-Tag specific antiserum; lane 3, hR6 antiserum) and of Ni-NTA affinity-purified (as described above in FIG. 8) glycosylated GlycoTag expressed in the presence of the protein glycosylation machinery of *C. jejuni* after digestion of amylose-agarose-affinity MalE-GlycoTag fusion protein with factor Xa protease (lane 2, His-Tag specific antiserum; lane 4, hR6 antiserum) is shown. Protein expression in *E. coli* DH5α, in the presence or the absence of the *C. jejuni* protein glycosylation operon on plasmid pACYC was induced with 0.1 mM IPTG for 18 h. For clarity, individual glycoprotein-specific signals are marked by a line on the side of the corresponding signal. The sizes of molecular weight markers in kDa are indicated on the left.

### DESCRIPTION

### Definitions

Unless defined otherwise, all technical and scientific terms used herein have the same meaning as commonly understood by one of ordinary skill in the art to which this invention belongs.

As used in the specification and claims, the singular forms "a", "an" and "the" include plural references unless the context clearly dictates otherwise.

The term "comprising" as used herein will be understood to mean that the list following is non-exhaustive and may or may not include any other additional suitable items, for example one or more further feature(s), component(s) and/or ingredient(s) as appropriate.

The term "nucleic acid sequence" (or nucleic acid molecule) refers to a DNA or RNA molecule in single or double stranded form, particularly a DNA encoding a protein or protein fragment according to the invention.

The terms "protein" or "polypeptide" are used interchangeably and refer to molecules consisting of a chain of amino acids, without reference to a specific mode of action, size, three-dimensional structure or origin. A "fragment" or "portion" of a protein may thus still be referred to as a "protein". A "peptide", as used herein, refers to a short polypeptide.

The term "toxoid" as used herein, refers to a bacterial toxin whose toxicity has been weakened, suppressed or inactivated either by chemical (e.g., formalin) or heat treatment, while other properties, typically immunogenicity, are maintained. As used herein, the term toxoid encompasses a protein or peptide fragment of a weakened, suppressed or inactivated toxin.

The term "gene" means a DNA sequence comprising a region (transcribed region), which is transcribed into an RNA molecule (e.g. an mRNA) in a cell, operably linked to suitable regulatory regions (e.g. a promoter). A gene may thus comprise several operably linked sequences, such as a promoter, a 5' leader sequence comprising e.g. sequences involved in translation initiation, a (protein) coding region (cDNA or genomic DNA) and a 3' non-translated sequence comprising e.g. transcription termination sites.

A "chimeric gene" (or recombinant gene) refers to any gene, which is not normally found in nature in a species, in particular a gene in which one or more parts of the nucleic acid sequence are not associated with each other in nature. For example, the promoter is not associated in nature with part or all of the transcribed region or with another regulatory region. The term "chimeric gene" is understood to include expression constructs in which a promoter or transcription regulatory sequence is operably linked to one or more coding sequences or to an antisense (reverse complement of the sense strand) or inverted repeat sequence (sense and antisense, whereby the RNA transcript forms double stranded RNA upon transcription).

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous and, where necessary to join two protein encoding regions, contiguous and in reading frame so as to produce a "chimeric protein". A "chimeric protein" is a protein composed of various protein "domains" (or motifs) which is not found as such in nature but which are joined to form a functional protein, which displays the functionality of the joined domains. A chimeric protein may also be a fusion protein of two or more proteins occurring in nature. The term "domain" as used herein means any part(s) or domain(s) of the protein with a specific structure or function that can be transferred to another protein for providing a new hybrid protein with at least the functional characteristic of the domain.

The term "target peptide", as used herein, refers to amino acid sequences that can target a protein to an intracellular organelle or location, such as periplasmic space, or to the extracellular space (secretion signal peptide). A nucleic acid sequence encoding a target peptide can be fused (in frame) to the nucleic acid sequence encoding the amino terminal end (N-terminal end) of the protein.

A "nucleic acid construct" or "vector" is herein understood to mean a man-made nucleic acid molecule resulting from the use of recombinant DNA technology and which can be used to deliver exogenous DNA into a host cell. Vectors can comprise further genetic elements to facilitate their use in molecular cloning, such as e.g. selectable markers, multiple cloning sites and the like (see below).

The term "GlycoTag peptide", as used herein, refers to a peptide having two or more copies of an *N*-linked glycosylation sequon, derived from the putative cytoplasmic protein Cj 1433c, which is naturally present in *C. jejuni* strain 11168. The GlycoTag peptide can be in a purified or isolated form and, optionally includes glycans attached thereto. The GlycoTag peptide glycosylated at at least one of the copies of the amino acid sequence as set for the in SEQ ID NO: 1 can be used alone or in combination with different proteins or peptides and can be incorporated into a chimeric protein or peptide for use, for example, in the development of antigen/antibody delivery systems, the preparation of vaccines, pharmaceuticals and diagnostic tests, and research into therapies for countering Campylobacter infections.

The above-described glycosylated GlycoTag peptide can be used to display any *N*-glycan, including those of a eukaryotic, bacterial and/or archaeal source, or *N-*glycan mimic generated from enzymes from any or all of the three sources. Moreover, the glycosylated GlycoTag peptide can be used to display any O-glycans, where only a threonine is required.

The GlycoTag peptide is derived from the N-terminal amino acid sequence of the protein Cj 1433c of *C. jejuni* strain 11168 (FIG. 3, SEQ ID NO: 31). This segment of the protein contains nine repeats of the amino acid sequence KIDLNNT (SEQ ID NO: 1), wherein each repeat contains an *N*-linked glycosylation sequon (DLNNT). Bioinformatic analyses (FIGS. 2A and 2B) have shown that the Cj 1433c protein containing the GlycoTag peptide is not secreted into the bacterial periplasmic space and is thus lacking one of the prerequisites for bacterial *N*-linked glycosylation in the native host. Moreover, use of the TMpred ("Prediction of Transmembrane Regions and Orientation") program (http://www.ch.embnet.org/software/TMPRED_form.html) and the TMHMM program for prediction of transmembrane helices in proteins (http://www.cbs.dtu.dk/services/TMHMM-2.0/) did not predict the presence of transmembrane domains in Cj 1433c. It was thus unexpected that the Cj 1433c protein would contain a sequence that is highly susceptible to glycosylation. In addition to these bioinformatics analyses, a whole glycoproteome analysis performed by Scott *et al.* (2010) could not detect the Cj 1433c protein as an *N*-linked glycoprotein in *C*. *jejuni* (Scott NE et al., 2010: Simultaneous glycan-peptide characterization using hydrophilic interaction chromatography and parallel fragmentation by CID, HCD and ETD-MS applied to the N-linked glycoproteome of Campylobacter jejuni. Mol Cell Proteomics, published on April 1, 2010 as Manuscript M000031-MCP201. Without wishing to be bound by theory, it is clear to one of skill in the art that these analyses show that the successful use of the GlycoTag peptide as an *N*-glycan acceptor peptide would be unlikely. Surprisingly, the present inventors have demonstrated that is not the case. In one embodiment, to allow for *N*-linked glycosylation in various host systems, the GlycoTag peptide can be modified to include a signal peptide that allows for export from the host cell. In one embodiment, the signal peptide allows for export to the periplasm of bacterial cells. In one embodiment, the GlycoTag peptide is fused to a pectate lyaseB leader sequence.

In one embodiment, the GlycoTag peptide comprises between 3 and 30 repeats of the amino acid sequence set forth in SEQ ID NO: 1.

In another embodiment, the GlycoTag peptide comprises between 3 and 30 repeats of the amino acid sequence set forth in SEQ ID NO: 1, wherein the repeats are separated from one another by up to 6 amino acids.

In another embodiment, the GlycoTag peptide comprises between 3 and 30 repeats of the amino acid sequence set forth in SEQ ID NO: 1, wherein the repeats are contiguous.

In another embodiment, the GlycoTag peptide comprises at least 9 repeats of the amino acid sequence set forth in SEQ ID NO: 1, wherein the repeats are separated from one another by up to 6 amino acids.

In another embodiment, the GlycoTag peptide comprises at least 9 repeats of the amino acid sequence set forth in SEQ ID NO: 1, wherein the repeats are contiguous.

In another embodiment, the GlycoTag peptide comprises about nine repeats of the amino acid sequence set forth in SEQ ID NO: 1. the nine repeats of SEQ ID NO: 1 are contiguous. In another embodiment, the nine repeats of SEQ ID NO: 1 are separated from one another by up to 6 amino acids.

It has now been determined that the GlycoTag peptide comprising about nine *N*-linked glycosylation sequons expose the full, or nearly full, complement of glycan structures, thereby effectively exposing a large number of glycan moieties within a single protein or peptide. Glycosylation of a GlycoTag peptide comprising 9 repeats of the amino acid sequence set forth in SEQ ID NO: 1 can generate a mixture of glycosylated peptides each containing from 0 - 9 glycans (as shown using SDS-PAGE analysis).

In one example, different glycan structures can be added to the GlycoTag peptide. For example, glycosylation can be achieved using different glycosylation pathways in *E. coli* to add different glycans onto the glycosylations sites. In a specific example, the use of inducible promoters associated with each gene cluster (for the different glycosylation pathways) so that none allowed to fully glycosylate the GlycoTag.

### Preparation of the GlycoTag peptide

As would be readily appreciated by a worker skilled in the art, the GlycoTag peptide and proteins comprising a GlycoTag peptide can be prepared using a variety of well known methods.

The GlycoTag peptide can be synthesized, in whole or in part, using solid-state peptide synthesis. Alternatively, the GlycoTag peptide can be prepared, in whole or in part, from isolated peptides obtained from natural sources.

The GlycoTag peptide can be prepared by recombinant expression of a vector comprising a nucleic acid sequence encoding the GlycoTag peptide, in a host cell that optionally comprises *N*-linked glycosylation machinery, using techniques known in the art. The host cell can be *E. coli* that has been transformed to express the *pgl* machinery of *C. jejuni* (Wacker M et al., 2002: N-linked glycosylation in Campylobacter jejuni and its functional transfer into E. coli. Science, 298(5599): 1790-3). The construction of chimeric genes and vectors for, preferably stable, introduction of GlycoTag peptide encoding nucleic acid sequences into host cells is generally known in the art. To generate a chimeric gene the nucleic acid sequence encoding a GlycoTag peptide is operably linked to a promoter sequence, suitable for expression in the host cells, using standard molecular biology techniques. The promoter sequence may already be present in a vector so that the GlycoTag peptide nucleic sequence is simply inserted into the vector downstream of the promoter sequence. The vector is then used to transform the host cells and the chimeric gene is inserted in the nuclear genome or into a plasmid and expressed there using a suitable promoter. In one embodiment a chimeric gene comprises a suitable promoter for expression in microbial cells (e.g., bacteria), operably linked thereto a nucleic acid sequence encoding a GlycoTag peptide or fusion protein as described herein.

In one embodiment, the GlycoTag peptide is fused to an affinity tag to facilitate purification by affinity chromatography. In one embodiment, the affinity tag is fused to the N-terminus of the GlycoTag peptide. In one embodiment, the affinity tag is fused to the C-terminus of the GlycoTag peptide. Of course, as one of skill in the art will appreciate, a wide variety of different affinity tags can be used, depending on the type of affinity purification contemplated. Specific examples of affinity tags that can be used to assist in purification of the GlycoTag peptide include, but are not limited to, epitope tags, chromatography tags, and, more specifically, a hexa-histidine tag (sometime referred to as a His-tag).

### Methods and Systems of Using the GlycoTag Peptide

The GlycoTag peptide can be used in a wide variety of different applications. The GlycoTag peptide can be used as an adjuvant in vaccine preparation. Of course, as one of skill in the art will appreciate, the GlycoTag peptide can be engineered to carry different N-glycans depending on the intended application.

In one embodiment, the GlycoTag peptide, when glycosylated with specific glycans, can be used as a glycan carrier in a vaccine which is for vaccination against zoonotic human pathogens such as *Campylobacter upsaliensis* (in cats and dogs), *Campylobacter coli* (in pigs) and *Campylobacter jejuni* (in chickens). As another example, which is also not meant to be limiting, the GlycoTag peptide glycosylated with specific glycans can be used in a vaccine to vaccinate humans against Travelers' Diarrhea (*Campylobacter jejuni*) and vaccinate cows and sheep to prevent infertility and abortions (*Campylobacter fetus venerealis* and *Campylobacter fetus fetus*).

In one embodiment, the GlycoTag peptide can be co-expressed recombinantly with an acceptor protein, a peptide or an antibody using techniques known in the art. The acceptor proteins, peptides and antibodies include, but are not limited to, toxins, toxoids, periplasmic proteins, antibodies and dentritic cell targeting antibodies. In one embodiment, the protein is AcrA. In one embodiment, the toxin can be selected from toxins expressed by *Bordetella pertussis, Clostridium tetani,* or *Corynebacterium diphtheria.* Of course, many other examples are possible. This can be particularly advantageous, since co-expression of the GlycoTag peptide with an acceptor protein, peptide or antibody can serve as the basis for the preparation of a range of useful products, including vaccines against Cambylobacter or other bacteria and vaccines having improved efficacy. In one example, which is not meant to be limiting, the GlycoTag peptide, when glycosylated with specific glycans and co-expressed with other proteins and peptide, can be used as a glycan carrier in a vaccine which is for vaccination against zoonotic human pathogens such as *Campylobacter upsaliensis* (in cats and dogs), *Campylobacter coli* (in pigs) and *Campylobacter jejuni* (in chickens). As another example, which is also not meant to be limiting, co-expression of the GlycoTag peptide glycosylated with specific glycans with other proteins and peptides can be used to prepare vaccines against Travelers' Diarrhea (*Campylobacter jejuni*) and against *Campylobacter fetus venerealis* and *Campylobacter fetus fetus.*

In one embodiment of the present invention, the glycosylated GlycoTag can be fused to an acceptor protein, a peptide or an antibody in order to direct or target the fusion to the periplasm of *E. coli* or of another expression organism. The multivalency of the GlycoTag has the advantage of attaching various glycan structures in multiple copies to a protein or peptide carrier in high density without having to engineer additional N-glycan acceptor sequences into target proteins. This can be particularly advantageous since it can allow for the recombinant expression of proteins and peptides that can display multiple N-glycan structures. As one of skill in the art will appreciate, a wide variety of carrier peptides and proteins are suitable for fusion with the GlycoTag. These include, but are not limited to, toxins, toxoids, periplasmic proteins, antibodies and dentritic cell targeting antibodies. In one embodiment, the protein is AcrA. In one embodiment, the toxin can be selected from toxins expressed by *Bordetella pertussis, Clostridium tetani,* or *Corynebacterium diphtheria.* Of course, many other examples are possible. This can be particularly advantageous, since the glycosylated GlycoTag fused to a carrier protein can serve as the basis for a range of useful products, including vaccines against Cambylobacter and vaccines having improved efficacy. The GlycoTag peptide can even be used in adjuvant-free compositions. In one example, which is not meant to be limiting, the GlycoTag, when glycosylated with specific glycans, can be used as a glycan carrier in a vaccine which is for vaccination against zoonotic human pathogens such as *Campylobacter upsaliensis* (in cats and dogs), *Campylobacter coli* (in pigs) and *Campylobacter jejuni* (in chickens). As another example, which is also not meant to be limiting, GlycoTag glycosylated with specific glycans can be used in a vaccine to vaccinate humans against Travelers' Diarrhea (*Campylobacter jejuni*) and vaccinate cows and sheep to prevent infertility and abortions (*Campylobacter fetus venerealis* and *Campylobacter fetus fetus*). As would be readily appreciated, the GlycoTag peptide can be used in the preparation of a vaccine for any pathogen immunogenic carbohydrate epitopes. In another non-limiting example, a suitably glycosylated GlycoTag peptide can be used in the manufacture of a vaccine against the oral pathogen, *C*. *rectus.*

As one of skill in the art will appreciate, there are many different ways in which the GlycoTag can be fused to a protein or peptide. The GlycoTag peptide can be fused directly or via a linker to a protein capable of being targeted to the periplasm of a selected expression vector organism such as *E. coli* that, in the presence of genes encoding protein glycosylation machinery, results in N-glycosylation of the respective sites on the GlycoTag.

In one embodiment, a GlycoTag fusion peptide or protein, or chimeric protein, is prepared from expression of a chimeric gene including a coding sequence for the GlycoTag peptide operably linked to a coding sequence for a second peptide or protein. Accordingly, also provided is a vector comprising such a chimeric gene and appropriate sequences for expression of the chimeric gene in a host organism.

In one embodiment, a fusion between the GlycoTag peptide and a protein can include an affinity tag to facilitate purification by affinity chromatography. Of course, as one of skill in the art will appreciate, a wide variety of different affinity tags can be used, depending on the type of affinity purification contemplated. Moreover, depending on the nature of the fusion product, the affinity tag can be placed at the N-terminus or at the C-terminus.

In one embodiment, the GlycoTag peptide can be modified to attach any lipid-linked saccharide expressed in the presence of the GlycoTag fusion protein.

In another embodiment, the glycosylated GlycoTag peptide is used in a diagnostic assay method or system. In one example of this embodiment, the GlycoTag peptide is engineered to display specific glycans characteristic of an infectious bacteria. The GlycoTag peptide can then be used in an immunological assay to detect, and optionally quantitate, antibodies directed to the infectious bacteria. In the immunological method, the glycosylated GlycoTag peptide is contacted with a biological sample from a subject suspected of bacterial infection and the mixture is incubated to allow binding complexes to form between the GlycoTag peptide and the antibodies present in the sample that specific for the displayed glycans. The binding complexes are then detected using standard methods, and optionally quantitated. As would be appreciated by a worker skilled in the art, the assay can be performed using a variety of well known techniques for immunological assays. In one embodiment, the glycosylated GlycoTag peptide is immobilized on a solid support prior to contact with the biological sample. The solid support can be, for example, microtitre plates, polymer beads, agarose beads, paramagnetic beads, or membranes.

In accordance with another aspect there is a provided an immunoassay kit comprising glycosylated GlycoTag peptides as described above and reagents for the detection of binding complexes formed with the glycosylated GlycoTag peptides and antibodies specific for bacterial antigens corresponding with the antigenic components of the glycosylated GlycoTag peptides. The immunoassay kit additionally includes instructions for use and may further include a container or other means for collecting a sample from a subject suspected of having a bacterial infection.

In order that the invention be more fully understood, the following examples are set forth. These examples are for illustrative purposes only and are not to be construed as limiting the scope of the invention in any way. Moreover, these examples are not intended to exclude equivalents and variations within the scope of the present invention, which are apparent to one skilled in the art.

### EXAMPLES

### EXAMPLE 1: Preparation of GlycoTag/AcrA fusion proteins

The glycoprotein AcrA of *Campylobacter fetus fetus* (AcrA^{Cff}), which naturally possesses two N-glycosylation sequons, was used as the protein carrier. A plasmid was constructed to express the *AcrA* gene of *C. fetus fetus* in *E. coli* BL21 using the pectate lyaseB (*pelB*) leader sequence on plasmid pET22b (Novagen) to direct the protein to the periplasmic space via the secretory (Sec) pathway. Therefore, primers AcrA^{Cff}-NcoI (5'- ATGGCCATGGATAATAAAAAATCAGCC-3' [SEQ ID NO: 3]) and AcrA^{Cff}-XhoI (5'-TTTCTCGAGTTTGCTTCCTTTGGCATCATTTATCG-3' [SEQ ID NO: 4]) were used to amplify nucleotides 67 to 1052 of the *AcrA* gene from chromosomal DNA from *C. fetus fetus* 82-20, while simultaneously introducing restriction sites (underlined) for *Nco*I and *Xho*I*.* The PCR product was inserted into plasmid pET22b, which had been digested with the same restriction enzymes. The AcrA protein is a periplasmic protein in the native host and it is also a target for *N*-linked glycosylation when expressed in *E. coli* BL21 in the presence of the *C. jejuni* protein glycosylation operon (after induction with 0.1 mM IPTG, Figure 7C, lane 1).

The GlycoTag peptide sequence was prepared as follows: The DNA sequence encoding the N-terminus of the Cj1433c protein [SEQ ID NO: 2], which is **CTCGAG**TTCATAAAAAATTTCAAGCAACATGAAAAAATTAAGATAGATCT TAATAATACAAAGATAGATCTTAATAATACAAAGATAGATCTTAATAATA CAAAGATAGATCTTAATAATACAAAGATAGATCTTAATAATACAAAGATA GATCTTAATAATACAAAGATAGATCTTAATAATACAAAGATAGATCTTAA TAATACAAAGATAGATCTTAATAATACAAAGATAGAATTATCGCAATTAA AAAAAGAGC**TCGAG** (the restriction sites for *Xho*I are shown in bold), was generated by PCR with gene-specific oligonucleotides (1433c-XhoI-1: 5'-AAA**CTCGAG**TTCATAAAAAATTTCAAGC-3' [SEQ ID NO: 5] and 1433c-XhoI-2: 5'-ATAT**CTCGAG**CTCTTTTTTTAATTGCG-3' [SEQ ID NO: 6]) from chromosomal DNA of the genome-sequenced strain *Campylobacter jejuni* 11168 (Parkhill J et al., 2000: The genome sequence of the food-borne pathogen Campylobacter jejuni reveals hyper-variable sequences. Nature, 403(6770):665-668). The digested DNA fragments were fused in frame to the N-terminus (construct 1, FIG. 4 [SEQ ID NO: 7]), to the C-terminus (construct 2, FIG. 4 [SEQ ID NO: 8]) and in between the lipoyl- and the α-hairpin domains (as described in Symmons M et al., 2009: The assembled structure of a complete tripartite bacterial multidrug efflux pump. PNAS, 106(17): 6893-6894) of the *C. fetus fetus* AcrA protein (AcrA^{Cff}) at nucleotide position 457 of the AcrA^{Cff} coding sequence (construct 3, FIG. 4 [SEQ ID NO: 9]). For construct 1, the above-described PCR product was introduced via the *Nco*I site. For construct 2, the above-described PCR product was introduced via the *Xho*I site. For construct 3, the above-described PCR product was introduced via the *Nhe*I site. The correct insertion of the PCR products into the respective sites was verified by DNA sequencing. The fusion proteins (constructs 1, 2, and 3) were expressed in an *E. coli* BL21 expression system as follows. At an OD₆₀₀ of 0.6 (after growth at 37°C), cultures were induced with 0.1 mM of IPTG and cells were grown for an additional 18 h at the same temperature. Cultures were cooled on ice for 15 min and cells were harvested by centrifugation (10 min, 10,000 rpm, 4°C), washed twice in one culture volume of ice-cold PBS buffer and finally re-suspended in 1/20 of the original culture volume. Cells were disrupted by sonication (3 times 1 minute pulses) and centrifuged for 30 min at 15,000 rpm at 4°C). His-tagged proteins were purified from the supernatants using The QiaExpressionist (Qiagen). Analyses of Ni-NTA affinity chromatography-purified proteins was followed by SDS-PAGE and Western Blot analyses with monoclonal antibodies specific against the Hexa-histidine tag (Santa Cruz Biotech Inc.). The Hexa-histidine tag-specific western blot showed that only construct 2 resulted in the overexpression of a protein with the correct size of approximately 55 kDa (Figure 5, lane 2). The deduced amino acid sequence of the N-(construct 1), the C-(construct 2) and interdomain (construct 3) AcrA^{Cff}-GlycoTag-His fusion proteins are shown in FIG. 6A, 6B and 6C, respectively.

### EXAMPLE 2: Expression of GlycoTag/AcrA construct 2 in E. coli BL21 (pgl+)

Expression in *E. coli* BL21 in the presence of the *C. jejuni* glycosylation machinery located on plasmid pACYC184 (pACYC (pgl-Cj)) (Wacker M et al., 2002: N-linked glycosylation in Campylobacter jejuni and its functional transfer into E. coli. Science, 298(5599): 1790-1793) resulted in soluble, periplasmic expression of the C-terminal AcrA-GlycoTag-His fusion protein. The fusion protein was purified by Ni-NTA affinity chromatography and was examined by western blotting with monoclonal antibodies specific against the Hexa-histidine tag (Santa Cruz Biotechnology Inc.) and *C. jejuni N*-glycan-specific antiserum (hR6). FIG. 7B and 7C show the western blots with hR6 and anti-Hexa-histidine (anti-His). A mixture of site occupancy occurs, as we observed fusion proteins with 1, 2, 3, 4, 5, 6, 7, 8, 9, 10 and 11 *N*-glycans attached. As shown in FIG. 7D, we could detect a chimeric glycoprotein with 11 *C. jejuni* heptasaccharides attached (2 natural sites of AcrA ^{Cff}, 9 sites of GlycoTag peptide). This result was unpredicted because it was proposed previously that a specific protein conformation would improve glycosylation efficiency while in the current example, the *N*-linked glycosylation sequons (DLNNT) were separated by only two amino acids (KI) in the GlycoTag peptide.

### EXAMPLE 3: Preparation GlycoTag/toxoid fusion proteins

The GlycoTag sequence of SEQ ID NO: 10, prepared as described in Example 1, was fused to the N-terminus and the C-terminus of the immunostimulating toxoid gene products of *Bordetella pertussis* (*ptxA,* N-terminus, nt 1 - 537), *Clostridium tetani* (*tet,* C-terminus, nt 2566 - 3945) and *Corynebacterium diphtheriae* NCTC 13129 (*tox_{Cd},* nt 79 - 654) (FIG. 8A). PCR products were generated with the following toxoid gene-specific oligonucleotides, while introducing restriction sites (*Nco*I, *Xho*I):

| | |
|---|---|
| ptxA-NcoI-1 | 5' AATATATACCATGGGTTGCACTCGGGCAATTCG-3 [SEQ ID NO: 11] |
| ptxA-XhoI-2 | 5'-ATATATCTCGAGCGTTACCCTGCGGATGTTTTCGG-3' [SEQ ID NO: 12] |
| tet-NcoI-1 | |
| tet-XhoI-2 | |
| tox-NcoI-1 | 5'-ATATATATCCATGGCTGCTGATGATGTTGTTGATTC-3' [SEQ ID NO: 15] |
| tox-XhoI-2 | 5'- ATATACTCGAGTCGCCTGACACGATTTCCTGCACAGG-3' [SEQ ID NO: 16] |

A plasmid described by Aminian *et al.* (2007) served as a template for the PCR reaction (Aminian et al., 2007: Expression and purification of a trivalent pertussis toxin-diphtheria toxin-tetanus toxin fusion protein in Escherichia coli. Protein Expr Purif, 51(2): 170-178). Obtained PCR products were introduced into the plasmid expressing the N-terminal or the C-terminal GlycoTag AcrA^{Cff}-His fusion proteins. Therefore, the *Xho*I restriction site closer to the Hexa-histidine encoding sequence and the *Nco*I site closer to the *pelB* leader peptide encoding sequence were deleted by quick change mutagenesis using the QuikChange® Mutagenesis Kit (Stratagene) with the following oligonucleotides:

| | |
|---|---|
| QC-XhoI-Del-1 | |
| QC-XhoI-Del-2 | 5'- CTCAGTGGTGGTGGTGGTGGTGCTCGATCTCTTTTTTTAATTGC-3' [SEQ ID NO: 18] |
| QC-NcoI-Del-1 | |
| QC-NcoI-Del-1 | |

The plasmids encoding either the N-terminal AcrA^{Cff}-GlycoTag-His or the C-terminal AcrA^{Cff}-GlycoTag-His fusion proteins served as templates.

Subsequently, the AcrA^{Cff} protein coding sequence was exchanged by the PCR-amplified toxin genes (as described above) and were inserted via *Nco*I*-Xho*I to create either the C- or the N-terminal Hexa-histidine-tagged GlycoTag-toxin fusion constructs.

Fusion protein expression, secretion into the periplasmic space of *E. coli* and N-glycosylation was analyzed as described above. Expression, affinity-tag purification (see above), and glycosylation in the heterologous *E. coli* protein glycosylation system (see above) could be demonstrated for the C-terminal pelB-toxC_{Cd}-GlycoTag-HexaHistidine construct (FIGS. 8C and 8D). Again, a mixture of site occupancy occurs, as fusion proteins were observed with 1, 2, 3, 4, 5, 6, 7, 8 and 9 N-glycans attached (FIGS. 8C and 8D). Interestingly, it appears that the N-glycan carrier peptide induces dimer formation in the fusion increasing the valency of the construct.

### EXAMPLE 4: Preparation of two GlycoTag peptides fused to a toxoid protein.

A second GlycoTag peptide of SEQ ID NO: 10 was attached to the ToxC_{Cd} fusion protein made in Example 3 to create a ToxC_{Cd}-(GlycoTag)₂ fusion protein (FIG. 9A). Therefore, a 258 bp gel-purified DNA fragment obtained after *Xho*I digestion of the C-terminal pelB-AcrA^{Cff}-GlycoTag-His fusion protein-encoding plasmid was inserted into the *Xho*I digested C-terminal pelB-ToxCcd-GlycoTag-His fusion protein-encoding plasmid. The orientation of the second GlycoTag-encoding DNA sequence was verified by DNA sequencing with the oligonucleotide hybridizing in the Hexa-histidine-encoding plasmid DNA sequence. Expression and glycosylation with the *N*-glycan of *C. jejuni* (in the presence of the *C. jejuni* protein glycosylation machinery-encoding plasmid) and Ni-NTA affinity chromatography was performed as described above. Analyses by western blotting with *N*-glycan-specific antiserum (hR6) resulted in a protein with up to 18 *C. jejuni N*-glycans attached (FIG. 9B). However, a mixture of site occupancy occurs, as we observed fusion proteins with 1, 2, 3, 4, 5, 6, 7, 8, 9, 10, 11, 12, 13, 14, 15, 16, 17 and 18 *N*-glycans attached.

### EXAMPLE 5: Preparation of GlycoTag/antibody fusion proteins

The sequence encoding for the GlycoTag peptide of SEQ ID NO: 10, prepared as described in Example 1, was fused at the C-terminal and N-terminal of a single chain antibody (scFv) that recognizes the DEC-205 receptor of dendritic cells (DCs) and that can be expressed in *E. coli* BL21 (FIG. 8B) as described above (Wang WW et al.: 2009: A versatile bifunctional dendritic cell targeting vaccine vector. Mol Pharm, 6(1): 158-172). Therefore, a PCR product was generated with specific oligonucleotides (Ab-1433-XhoI, 5'-ATATCTCGAGGCGGCCGCAAGCTGAGGAGACTGTGACCA TGACTCC-3' [SEQ ID NO: 21], pET-PstI 5'-AATTCTGCAGTAATTTTGTTTAACTTTAAGAAGG-3' [SEQ ID NO: 22] together with plasmid pWET7; Ab-1433-NcoI, 5'- ATATATATCCATGGGAGGTGGCGGATCAGAAGTGAAGC-3' [SEQ ID NO: 23] and pET-KpnI ATATGGTACCTTAGCAGCCGGATCTCAGTGG-3' [SEQ ID NO: 24] together with plasmid pWET5) that amplify the scFv-DEC205 gene from plasmid pWET7 (to create the C-terminal scFvDEC205-GlycoTag fusion) and from plasmid pWET5 (to create the N-terminal scFvDEC205-GlycoTag fusion) (Wang WW et al., 2009: A versatile bifunctional dendritic cell targeting vaccine vector. Mol Pharm, 6(1): 158-172). A strategy similar to that described above for the creation of the toxoid-GlycoTag fusion proteins was applied to exchange the AcrA ^{Cff} encoding DNA sequence with the scFvDEC205-encoding DNA sequence. We could express (as described above), affinity-tag purify (as described above), and glycosylate the C-terminal pelB-scFvDEC205-GlycoTag-HexaHistidine construct in the heterologous *E*. *coli* protein glycosylation system (FIGS. 8E and 8F). Again, a mixture of site occupancy occurs, as we observed fusion proteins with 1, 2, 3, 4, 5, 6, 7, 8 and 9 *N-*glycans attached.

The purpose of targeting the N-glycosylated GlycoTag peptide directly to dendritic cells (DC) is to reduce the amount of antigen required and also to eliminate the adjuvant requirement for immune stimulation. DCs are the antigen presenting cells of the immune system that play a critical role in innate and adaptive immune responses, especially in priming and activating T cell and B cell immunity.

### EXAMPLE 6: Immunization of mice with GlycoTag fusion proteins

Six groups each with five C57BL/6 female mice were immunized with purified GlycoTag fusion proteins as set out in Table 1.

**Table 1**

| **Group** | **Immunization material** | **Day 0** | **Day 12** | **Day 21** |
|---|---|---|---|---|
| 1 | Control | PBS | PBS | Serum (Humoral) |
| 2 | Glycosylated DEC205-GlycoTag and Anti-CD40 mAb | 1 µg | 1 µg | Serum (Humoral) |
| | | 25 µg | 0 | |
| 3 | Glycosylated Acr^{Cff}-GlycoTag and Anti-CD40 mAb | 1 µg | 1 µg | Serum (Humoral) |
| | | 25 µg | | |
| 4 | Glycosylated ToxC_{Cd}-GlycoTag and Anti-CD40 mAb | 1 µg | 1 µg | Serum (Humoral) |
| | | 25 µg | 0 | |
| 5 | Glycosylated Acr^{Cff} and Anti-CD40 mAb | 1 µg | 1 µg | Serum (Humoral) |
| | | 25 µg | 0 | |
| 6 | Unglycosylated AcrA^{Cff} and Anti-CD40 mAb | 1 µg | 1 µg | Serum (Humoral) |
| | | 25 µg | 0 | |

The humoral (antibody) response followed in mouse blood serum (isolated using standard procedure) of groups 1 to 6 showed that *N*-glycan-specific antibodies were produced in groups 2, 3, 4, and 5 in the order 2 > 4 > 3 > 5. No humoral response against the N-glycan was observed in groups 1 and 6 (FIG. 10).

### EXAMPLE 7: Immunization of mice with purified GlycoTag

Seven groups each with five C57BL/6 female mice were immunized with purified GlycoTag or purified GlycoTag fusion proteins as set out in Table 2.

**Table 2**

| | **Immunization material** | **Day 0 Freund's complete** | **Day 12 Freund's incomplete** | **Day 21** | **Day 28 Freund's incomplete** | **Day 35** |
|---|---|---|---|---|---|---|
| Gr 1 | control | PBS | PBS | Serum (Humoral) | PBS | Serum (Humoral) |
| Gr 2 | Unglycosylated GlycoTag | 1 µg | 1 µg | Serum (Humoral) | 1 µg | Serum (Humoral) |
| Gr 3 | Glycosylated GlycoTag | 2 µg = 1 µg (sugar) | 2 µg = 1 µg (sugar) | Serum (Humoral) | 2 µg = 1 µg (sugar) | Serum (Humoral) |
| Gr 4 | Unglycosylated ToxC_{Cd}-GlycoTag | 30 µg | 30 µg | Serum (Humoral) | 30 µg | Serum (Humoral) |
| Gr 5 | Glycosylated ToxC_{Cd}-GlycoTag-(app. 3-9 Cj glycans) | 7 µg = 1 µg (sugar) | 7 µg = 1 µg (sugar) | Serum (Humoral) | 7 µg = 1 µg (sugar) | Serum (Humoral) |
| Gr 6 | Unglycosylated ToxC_{Cd}-(GlycoTag)2 | 3.5 µg | 3.5 µg | Serum (Humoral) | 3.5 µg | Serum (Humoral) |
| Gr 7 | Glycosylated ToxC_{Cd}-(GlycoTag)2 (appr 5-18 Cj glycans) | 3.5 µg = 1 µg (sugar) | 3.5 µg = 1 µg (sugar) | Serum (Humoral) | 3.5 µg = 1 µg (sugar) | Serum (Humoral) |

The humoral (antibody) response followed in mouse blood serum (isolated using standard procedure) of groups 1 to 6 from day 21 (FIG 11A) and from day 35 (FIG 11B) showed that *N*-glycan-specific antibodies were produced in groups 3, 5, and 7 in the order 3 > 5 = 7. No significant humoral response against the *N-*glycan was observed in groups 1, 2, 4 and 6.

Interestingly, these results demonstrate that use of the GlycoTag alone induced a stronger antibody response than when it was conjugated to a toxoid.

### EXAMPLE 8: Immunization of chicken with GlycoTag fusion proteins.

Eight groups each with eight chickens were immunized with purified GlycoTag fusion proteins as follows: At day 0, cecal swabs of 10% of all birds (5 birds were randomly selected) showed no colonization with Campylobacter when plated on selective Karamali agar plates after a 2 day incubation period under microaerobic conditions. At day 7, a pre-bleed of 500 µl was taken from each chicken followed by the 1^{st} antimicrobial treatment with 5 µg of antigen in a total volume of 300 µl for each bird (Table 3) that was administered intramuscularly (IM) by injecting 2-times 150 µl at different sites (using Freund's complete adjuvant in a 1:1 ratio with the antigen that was prepared in sterile PBS) or by oral (O) administration. At day 21, a test bleed was carried out followed by the second antimicrobial treatment with the same antigen formulation but replacing Freund's complete adjuvant with Freund's incomplete adjuvant. At day 28, chickens within groups 2-8 were gavaged (challenged) with *Campylobacter jejuni* (1.5x10*8 cells prepared in sterile PBS). At day 35, the chicken were euthanized, blood samples were taken from each bird (final bleed) and the colonization levels were determined by analyzing the amount of campylobacter cells in the cecum of each chicken by plating serial 10-fold serial dilutions (10*-2 to 10*-10) of the cecal contents on selective Karmali agar plates. After a 2 day incubation period under microaerobic conditions the formation of Campylobacter colonies was recorded (Figure 12A). Reduced *Campylobacter jejuni* colonization levels were obtained after vaccination with ToxC-GT1, 1-9 *C. jejuni* (Cj)-glycans (IM) and microencapsulated (MC) ToxC-GT1, 1-9Cj-glycans (O). The humoral (antibody) response followed in blood sera (isolated using standard procedures) showed that *N*-glycan-specific antibodies were produced in chickens from groups 4 and 5. No humoral response against the N-glycan was observed in pooled sera of chicken from group 1 (FIG. 12B)

**Table 3**

| | **Antimicrobial treatment (5 µg of antigen)** | **Challenge with Campylobacter (1.5x10*8 cfu)** |
|---|---|---|
| Group 1 | PBS | no |
| Group 2 | PBS | yes |
| Group 3 | ToxC-GT1 (no glycan) (IM) | yes |
| Group 4 | ToxC-GT1, 1-9Cj-glycans (IM) | yes |
| Group 5 | Micro-encapsulated (MC) ToxC-GT1, appr. 1-9 Cj-glycans (O) | yes |
| Group 6 | ToxC-2GlycoTags, appr 5-18 Cj glycans (IM) | yes |
| Group 7 | ToxC-GT1, appr. 1-9 Cj-glycans (O) | yes |
| Group 8 | Microcapsules, no protein (O) | yes |

Microencapsulated antigens (MC) using chitosan (CS)-based nanoparticles were prepared by polyelectrolyte complexation of positively charged CS with the polyanion tripolyphosphate (TPP)) as described (Makhlof A., Tozuka Y., and Takeuchi H., 2011: Design and evaluation of novel pH-sensitive chitosan nanoparticles for oral insulin delivery, European Journal of Pharmaceutical Sciences, 42(5), 445-451).

### EXAMPLE 9: Dose-dependent immunization of chicken with GlycoTag fusion protein.

Eight groups each with eight chickens were immunized with purified GlycoTag fusion ToxC-GT1 that carries 1-9Cj-glycans by intramuscular injection as follows: 8 groups each with 8 chickens were immunized with purified GlycoTag fusion proteins as described in (Table 4). At day 0, cecal swabs of 10% of all birds (5 birds were randomly selected) showed no colonization with Campylobacter when plated on Karamali agar plates after a 2 day incubation period under microaerobic conditions. At day 7, a pre-bleed of 500 µl was taken from each chicken followed by the 1^{st} antimicrobial treatment of antigen in a total volume of 300 µl for each bird that was administered intramuscularly (IM) by injecting 2-times 150 µl at different sites (using Freund's complete adjuvant in a 1:1 ration with the antigen prepared in sterile PBS) At day 21, a test bleed was carried out followed by the second antimicrobial treatment with the same antigen formulation but replacing Freund's complete adjuvant with Freund's incomplete adjuvant. At day 28, chickens within groups 2-8 were gavaged (challenged) with *Campylobacter jejuni* wild-type cells (10*2 or 10*6). At day 35, the chicken were euthanized, blood samples were taken from each bird (final bleed) and the colonization levels were determined by analyzing the amount of campylobacter cells in the cecum of each chicken by plating serial 10-fold serial dilutions (10*-2 to 10*-10) of the cecal contents on selective Karmali agar plates. After a 2 day incubation period under microaerobic conditions the formation of Campylobacter colonies was recorded (Figure 13).

**Table 4**

| | **Antimicrobial treatment** | **Amount of antigen (µg)** | **Challenge with *Campylobacter jejuni* cells (cfu)** |
|---|---|---|---|
| Group 1 | PBS | - | - |
| Group 2 | PBS | - | 10*2 |
| Group 3 | PBS | - | 10*6 |
| Group 4 | ToxC-GT1, 1-9 Cj-glycans (IM) | 5 | 10*2 |
| Group 5 | ToxC-GT1, 1-9 Cj-glycans (IM) | 100 | 10*2 |
| Group 6 | ToxC-GT1, 1-9 Cj-glycans (IM) | 5 | 10*6 |
| Group 7 | ToxC-GT1, 1-9 Cj-glycans (IM) | 100 | 10*6 |
| Group 8 | ToxC-GT1 (no glycan) (IM) | 100 | 10*6 |

Reduced Campylobacter colonization levels were obtained after vaccination with ToxC-GT1, 1-9Cj-glycans (IM) in a Campylobacter challenge dose dependent manner).

### EXAMPLE 10: Preparation of purified MalE-GlycoTag fusion protein

A PCR fragment containing the GlycoTag peptide-encoding DNA (SEQ ID NO: 10) was amplified with oligonucleotides CS492 (5'-AAAATAAATATAGGACAACCGGTTCAGG -3' [SEQ ID NO: 25]) and CS 491 (5'- TATCTCGAGTCACTCTTTTTTTAATTGCG -3' [SEQ ID NO: 26]) using the plasmid AcrA^{Cff}-GlycoTag-His as the template, with the *Xho*I site deleted using the Quick Change Mutagenesis Kit (Stratagene). The PCR product was digested with *Xho*I*,* and ligated with *Sal*I-digested p2x plasmid (New England Biotech). The correct orientation of the insert was confirmed by sequencing of the products with CS491.

Transcription of the MalE-GlycoTag fusion protein from the IPTG-inducible ptac promoter located on plasmid pMAL-p2X (New England Biolabs) in *E*. *coli* results in periplasmic expression of a MalE-GlycoTag fusion protein (SEQ ID NO: 27).

After over-expression in *E. coli,* the MalE-GlycoTag fusion protein can be purified using a combination of i) amylose affinity chromatography; ii) proteolytic cleavage of the MalE-GlycoTag fusion protein (e.g., with factor Xa); and/or iii) size exclusion chromatography. In all cases, the GlycoTag peptide is obtained in pure form.

### EXAMPLE 11: Preparation of GlycoTag using a MalE-GlycoTag-His fusion protein

A PCR fragment containing the GlycoTag peptide-encoding DNA (SEQ ID NO: 10) was amplified with oligonucleotides CS492 (5'-AAAATAAATATAGGACAACCGGTTCAGG -3' [SEQ ID NO: 28]) and CS 494 (5'- AATTTAAGCTTTGTTAGCAGCCGGATCTCAGTGG-3' [SEQ ID NO: 29]) using plasmid AcrACff-GT-His-(*Xho*I deleted by quick change mutagenesis) as template. The PCR product was digested with *Xho*I and *Hind*III and ligated with *Sal*I-*Hind*III digested plasmid pMAL-p2X.

Transcription of the MalE-GlycoTag-His fusion protein from the IPTG-inducible ptac promoter located on plasmid pMAL-p2x in *E. coli* results in periplasmic expression of a MalE-GlycoTag-His fusion protein (SEQ ID NO: 30, FIG. 14)

After overexpression in *E. coli* in the presence or the absence of the *Campylobacter jejuni* glycosylation operon, the MalE-GlycoTag fusion protein can be purified using a combination of i) amylose affinity chromatography; ii) proteolytic cleavage of the MalE-GlycoTag fusion protein (*e.g.*, with factor Xa, FIG.15); and/or iii) size exclusion chromatography, Ni-NTA-affinity chromatography, or electro-elution after separation on a gel. In all cases, the GlycoTag peptide is obtained in pure form.

### EXAMPLE 12: GlycoTag as vaccine

The GlycoTag peptide can be used as an oligosaccharide carrier peptide in immunogenicity studies against the attached oligosaccharide. The glycosylated GlycoTag can be fused to an immuno-stimulating protein (toxin) that will eliminate the need for an additional adjuvant. The glycosylated GlycoTag-peptide can be administered in combination with the preferred/standardized adjuvant for various species/applications. If necessary, purely glycosylated GlycoTag peptide can be obtained after chemical or proteolytic digestion of multiples of the glycosylated GlycoTag that is present as a mixture of GlycoTag peptide with none or 1, 2, 3, 4, 5, 6, 7, 8, or 9 sugars attached (as seen in FIG. 7D).

The glycosylation of the GlycoTag peptide yields protection from chemical or proteolytic cleavage. The oligosaccharide that is attached to the amino acid repeats (SEQ ID NO: 1) prevents cleavage by the protease trypsin. This has been confirmed by observing that non-glycosylated or incompletely glycosylated GlycoTag peptide were cleaved, whereas the glycosylated portion of the GlycoTag peptide remained intact, following exposure to protease (results not shown).

All publications, patents and patent applications mentioned in this Specification are indicative of the level of skill of those skilled in the art to which this invention pertains.

The invention being thus described, it will be obvious that the same may be varied in many ways. All such modifications as would be obvious to one skilled in the art are intended to be included within the scope of the following claims.

## Claims

1. A peptide comprising at least two repeats, preferably nine repeats, of the amino acid sequence as set forth in SEQ ID NO: 1, wherein the repeats of the amino acid sequence as set forth in SEQ ID NO: 1 are contiguous or separated by no more than 6 amino acids, wherein the peptide is glycosylated at at least one of the repeats of the amino acid sequence as set forth in SEQ ID NO: 1.

2. The peptide of claim 1, wherein the peptide further comprises a signal peptide that allows for periplasmic secretion.

3. A chimeric protein comprising at least one peptide as defined in any one of claims 1 - 2 and a second peptide or a protein, wherein the second peptide or protein preferably comprises a peptide or protein derived from, or native to, a Campylobacter, or is AcrA, a toxin, toxoid, an antibody or a periplasmic protein.

4. A method for producing a chimeric protein comprising multiple glycosylation sites, comprising:
engineering a chimeric gene comprising a nucleic acid sequence encoding a peptide comprising at least two repeats, preferably nine repeats, of the amino acid sequence as set forth in SEQ ID NO: 1, wherein the repeats of the amino acid sequence as set forth in SEQ ID NO: 1 are contiguous or separated by no more than 6 amino acids, wherein the peptide may further comprise a signal peptide that allows for periplasmic secretion, said nucleic acid sequence being operably linked, directly or via a linker polynucleotide, to a nucleic acid sequence encoding a second peptide or a protein, which second peptide or protein is preferably AcrA, a toxin, a toxoid, an antibody or a periplasmic protein; and
expressing the chimeric gene in a host.

5. The method of claim 4, wherein the second protein comprises an antibody.

6. The method of claim 4, wherein the second protein or peptide is derived from, or native to, a Campylobacter.

7. The chimeric protein of claim 3 or the method of claim 4, wherein the toxin or toxoid is derived from *Bordetella pertussis, Clostridium tetani,* or *Corynebacterium diphtheriae* and wherein the antibody targets a dendritic cell.

8. The method of any one of claims 4 - 7, wherein the host is *E. coli.*

9. The method of any one of claims 4 - 8, further comprising glycosylating the chimeric protein with a plurality of N-linked glycans, wherein the N-linked glycans are preferably derived from *C. jejuni.*

10. Use of the peptide defined in any one of claims 1 - 2 to display N- glycans of eukaryotic, bacterial and/or archaeal origin.

11. Use of the peptide defined in claim 1 or the chimeric protein defined in claim 3 as a glycan carrier, for example, in a vaccine, optionally in combination with a second protein or peptide, or the chimeric protein defined in claim 3 for the preparation of a vaccine or pharmaceutical.

12. A vaccine comprising a peptide as defined in claim 1 or a chimeric protein as defined in claim 3, wherein the vaccine is for:
vaccination against a zoonotic human pathogen, such as *Campylobacter upsaliensis* in cats and dogs, *Campylobacter coli* in pigs or *Campylobacter jejuni* in chickens; Travelers' Diarrhea caused by *Campylobacter jejuni;* or *Campylobacter rectus;* or
vaccination of cows or sheep to prevent infertility and abortions caused by *Campylobacter fetus venerealis* or *Campylobacter fetus fetus.*

13. A method for diagnosing a bacterial infection in a subject comprising:
(i) contacting a sample which has been obtained from the subject with peptides as defined in claim 1, wherein the glycans are representative of glycans present on bacterial antigens; and
(ii) detecting binding complexes formed by binding of the peptides as defined in claim 1 by the antibodies specific for bacterial antigens, wherein the presence of binding complexes is indicative of bacterial infection in the subject,
and wherein the bacterial antigens are preferably from *Bordetella pertussis; Clostridium tetani; Corynebacterium diphtheriae;* a zoonotic human pathogen, such as *Campylobacter upsaliensis, Campylobacter coli* or *Campylobacter jejuni*; *Campylobacter rectus*; *Campylobacter fetus venerealis* or *Campylobacter fetus fetus.*

14. The method according to claim 13, wherein the peptides as defined in claim 1 are immobilized on a solid support, wherein the solid support preferably is the surface of a microtitre plate, polymer beads, agarose beads, paramagnetic beads, or membranes.

15. An immunoassay kit for diagnosing a bacterial infection in a subject, the kit comprising:
(a) a mixture of one or more peptides as defined in claim 1, wherein each peptide comprises at least one glycan that is a bacterial antigen; and
(b) instructions for contacting said mixture with a biological sample from the subject and monitoring for formation of binding complexes formed by binding of the peptides as defined in claim 1 by antibodies in the sample that are specific for bacterial antigens.

## Patentansprüche

1. Peptid, umfassend mindestens zwei Wiederholungen, bevorzugt neun Wiederholungen, von der Aminosäuresequenz wie in SEQ ID NO: 1 angegeben, wobei die Wiederholungen der Aminosäuresequenz wie in SEQ ID NO: 1 angegeben zusammenhängend oder durch nicht mehr als 6 Aminosäuren getrennt sind, wobei das Peptid an mindestens einer der Wiederholungen der Aminosäuresequenz wie in SEQ ID NO: 1 angegeben glykosyliert ist.

2. Peptid nach Anspruch 1, wobei das Peptid ferner ein Signalpeptid, das periplasmatische Sekretion ermöglicht, umfasst.

3. Chimäres Protein, umfassend mindestens ein Peptid, wie in einem der Ansprüche 1 - 2 definiert, und ein zweites Peptid oder ein Protein, wobei das zweite Peptid oder Protein bevorzugt ein Peptid oder Protein abgeleitet von oder nativ zu einem Campylobacter umfasst, oder AcrA, ein Toxin, Toxoid, ein Antikörper oder ein periplasmatisches Protein ist.

4. Verfahren zur Herstellung eines chimären Proteins, umfassend mehrere Glykosylierungsstellen, umfassend:
gentechnisches Herstellen eines chimären Gens, umfassend eine ein Peptid kodierende Nukleinsäuresequenz, umfassend mindestens zwei Wiederholungen, bevorzugt neun Wiederholungen von der Aminosäuresequenz wie in SEQ ID NO: 1 angegeben, wobei die Wiederholungen der Aminosäuresequenz wie in SEQ ID NO: 1 angegeben zusammenhängend oder durch nicht mehr als 6 Aminosäuren getrennt sind, wobei das Peptid ferner ein Signalpeptid, das periplasmatische Sekretion ermöglicht, umfassen kann, die Nukleinsäuresequenz funktionell direkt oder über ein Linker-Polynukleotid mit einer Nukleinsäuresequenz verbunden ist, kodierend ein zweites Peptid oder ein Protein, wobei das zweite Peptid oder Protein bevorzugt AcrA, ein Toxin, ein Toxoid, ein Antikörper oder ein periplasmatisches Protein ist; und
Exprimieren des chimären Gens in einem Wirt.

5. Verfahren nach Anspruch 4, wobei das zweite Protein einen Antikörper umfasst.

6. Verfahren nach Anspruch 4, wobei das zweite Protein oder Peptid abgeleitet von oder nativ zu einem Campylobacter ist.

7. Chimäres Protein nach Anspruch 3 oder Verfahren nach Anspruch 4, wobei das Toxin oder Toxoid von *Bordetella pertussis, Clostridium tetani,* oder *Corynebacterium diphtheriae* abgeleitet ist, und wobei der Antikörper auf eine dendritische Zelle abzielt.

8. Verfahren nach einem der Ansprüche 4 - 7, wobei der Wirt *E. coli* ist.

9. Verfahren nach einem der Ansprüche 4 - 8, ferner umfassend Glykosylieren des chimären Proteins mit einer Vielzahl von N-verknüpften Glykanen, wobei die N-verknüpften Glykane bevorzugt von *C. jejuni* abgeleitet sind.

10. Verwendung des Peptids, definiert in einem der Ansprüche 1 - 2, um N-Glykane von eukaryotischem, bakteriellem und/oder archaealem Ursprung anzuzeigen.

11. Verwendung des Peptids, definiert in Anspruch 1, oder des chimären Proteins, definiert in Anspruch 3, als einen Glykanträger, zum Beispiel in einem Impfstoff, optional in Kombination mit einem zweiten Protein oder Peptid, oder des chimären Proteins, definiert in Anspruch 3, zur Herstellung eines Impfstoffs oder eines Arzneimittels.

12. Impfstoff, umfassend ein Peptid wie in Anspruch 1 definiert, oder ein chimäres Protein, wie in Anspruch 3 definiert, wobei der Impfstoff für:
Impfung gegen ein zoonotisches Humanpathogen, wie
*Campylobacter upsaliensis* in Katzen und Hunden, *Campylobacter coli* in Schweinen oder *Campylobacter jejuni* in Hühnern; Durchfälle bei Reisenden, verursacht durch *Campylobacter jejuni;* oder *Campylobacter rectus;* oder
Impfung von Kühen oder Schafen, um Infertilität und Aborte, verursacht durch *Campylobacter fetus venerealis* oder *Campylobacter fetus fetus,* zu verhindern, ist.

13. Verfahren zur Diagnose einer bakteriellen Infektion in einem Subjekt, umfassend:
(i) Kontaktieren einer Probe, die von dem Subjekt erhalten worden ist, mit Peptiden, wie in Anspruch 1 definiert, wobei die Glykane repräsentativ für auf bakteriellen Antigenen vorhandene Glykane sind; und
(ii) Detektieren bindender Komplexe, gebildet durch Binden der Peptide, wie in Anspruch 1 definiert, durch die für bakterielle Antigene spezifischen Antikörper, wobei das Vorhandensein von bindenden Komplexen auf bakterielle Infektion in dem Subjekt hinweist,
und wobei die bakteriellen Antigenen bevorzugt von *Bordetella pertussis; Clostridium tetani*; *Corynebacterium diphtheriae*; einem zoonotischen Humanpathogen, wie etwa *Campylobacter upsaliensis, Campylobacter coli* oder *Campylobacter jejuni*; *Campylobacter rectus*; *Campylobacter fetus venerealis* oder *Campylobacter fetus fetus* sind.

14. Verfahren nach Anspruch 13, wobei die Peptide, wie in Anspruch 1 definiert, auf einer festen Unterlage immobilisiert sind, wobei die feste Unterlage bevorzugt die Oberfläche von einer Mikrotiterplatte, Polymerkügelchen, Agarosekügelchen, paramagnetischer Kügelchen, oder Membranen ist.

15. Immunoassaykit zur Diagnose einer bakteriellen Infektion in einem Subjekt, das Kit umfassend:
(a) ein Gemisch von einem oder mehreren Peptiden, wie in Anspruch 1 definiert, wobei jedes Peptid mindestens ein Glykan, das ein bakterielles Antigen ist, umfasst; und
(b) Anweisungen zum Inkontaktbringen der Mischung mit einer biologischen Probe von dem Subjekt, und Überwachen auf Bildung von bindenden Komplexen, gebildet durch Binden der Peptide, wie in Anspruch 1 definiert, durch Antikörper in der Probe, die spezifisch für bakterielle Antigene sind.

## Revendications

1. Peptide comprenant au moins deux répétitions, de préférence neuf répétitions, de la séquence d'acides aminés telle que décrite dans SEQ ID NO: 1, où les répétitions de la séquence d'acides aminés telle que décrite dans SEQ ID NO: 1 sont contiguës ou séparées par 6 acides aminés au maximum, où le peptide est glycosylé à au moins une des répétitions de la séquence d'acides aminés telle que décrite dans SEQ ID NO: 1.

2. Peptide selon la revendication 1, où le peptide comprend en outre un peptide signal qui permet une sécrétion périplasmique.

3. Protéine chimérique comprenant au moins un peptide tel que défini dans l'une quelconque des revendications 1 à 2 et un(e) second(e) peptide ou protéine, où le/la second(e) peptide ou protéine comprend de préférence un peptide ou une protéine dérivé(e), ou natif/native, d'une Campylobacter, ou est AcrA, une toxine, une anatoxine, un anticorps ou une protéine périplasmique.

4. Procédé de production d'une protéine chimérique comprenant de multiples sites de glycosylation, comprenant :
l'ingénierie d'un gène chimérique comprenant une séquence d'acide nucléique codant pour un peptide comprenant au moins deux répétitions, de préférence neuf répétitions, de la séquence d'acides aminés telle que décrite dans SEQ ID NO: 1, où les répétitions de la séquence d'acides aminés telle que décrite dans SEQ ID NO: 1 sont contiguës ou séparées par 6 acides aminés au maximum, où le peptide peut en outre comprendre un peptide signal qui permet une sécrétion périplasmique, ladite séquence d'acide nucléique étant en liaison fonctionnelle, directement ou via un polynucléotide de liaison, avec une séquence d'acide nucléique codant pour un(e) second(e) peptide ou protéine, lequel/laquelle second(e) peptide ou protéine est de préférence AcrA, une toxine, une anatoxine, un anticorps ou une protéine périplasmique ; et
l'expression du gène chimérique dans un hôte.

5. Procédé selon la revendication 4, dans lequel la seconde protéine comprend un anticorps.

6. Procédé selon la revendication 4, dans lequel le/la second(e) protéine ou peptide est dérivé(e), ou natif/native, d'une Campylobacter.

7. Protéine chimérique selon la revendication 3 ou procédé selon la revendication 4, où la toxine ou l'anatoxine est dérivée de *Bordetella pertussis, Clostridium tetani,* ou *Corynebacterium diphtheriae,* et où l'anticorps cible une cellule dendritique.

8. Procédé selon l'une quelconque des revendications 4 à 7, dans lequel l'hôte est E. *coli.*

9. Procédé selon l'une quelconque des revendications 4 à 8, comprenant en outre la glycosylation de la protéine chimérique avec une pluralité de glycanes N-liés, où les glycanes N-liés sont de préférence dérivés de *C. jejuni.*

10. Utilisation du peptide défini dans l'une quelconque des revendications 1 à 2 pour présenter des N-glycanes d'origine eucaryote, bactérienne et/ou d'archée.

11. Utilisation du peptide défini dans la revendication 1 ou de la protéine chimérique définie dans la revendication 3 en tant que transporteur de glycanes, par exemple, dans un vaccin, facultativement en combinaison avec un(e) second(e) protéine ou peptide, ou de la protéine chimérique définie dans la revendication 3 pour la préparation d'un vaccin ou d'un produit pharmaceutique.

12. Vaccin comprenant un peptide tel que défini dans la revendication 1 ou une protéine chimérique telle que définie dans la revendication 3, où le vaccin est destiné à :
une vaccination contre un agent pathogène humain zoonotique, tel que *Campylobacter upsaliensis* chez les chats et les chiens, *Campylobacter coli* chez les porcs ou *Campylobacter jejuni* chez les poulets ; la diarrhée du voyageur provoquée par *Campylobacter jejuni* ; ou *Campylobacter rectus* ; ou
une vaccination de vaches ou de moutons pour la prévention de l'infertilité et des avortements provoqués par *Campylobacter fetus venerealis* ou *Campylobacter fetus fetus.*

13. Procédé de diagnostic d'une infection bactérienne chez un sujet comprenant :
(i) la mise en contact d'un échantillon qui a été obtenu à partir du sujet avec des peptides tels que définis dans la revendication 1, où les glycanes sont représentatifs de glycanes présents sur des antigènes bactériens ; et
(ii) la détection de complexes de liaison formés par la liaison des peptides tels que définis dans la revendication 1 par les anticorps spécifiques pour les antigènes bactériens, où la présence des complexes de liaison est indicative d'une infection bactérienne chez le sujet,
et dans lequel les antigènes bactériens sont de préférence de *Bordetella pertussis* ; *Clostridium tetani; Corynebacterium diphtheriae ;* un agent pathogène humain zoonotique, tel que *Campylobacter upsaliensis, Campylobacter coli* ou *Campylobacter jejuni ; Campylobacter rectus ; Campylobacter fetus venerealis* ou *Campylobacter fetus fetus.*

14. Procédé selon la revendication 13, dans lequel les peptides tels que définis dans la revendication 1 sont immobilisés sur un support solide, où le support solide est de préférence la surface d'une plaque de microtitration, des billes polymériques, des billes d'agarose, des billes paramagnétiques, ou des membranes.

15. Kit de dosage immunologique pour diagnostiquer une infection bactérienne chez un sujet, le kit comprenant :
(a) un mélange d'un ou de plusieurs peptides tels que définis dans la revendication 1, où chaque peptide comprend au moins un glycane qui est un antigène bactérien ; et
(b) des instructions pour mettre en contact ledit mélange avec un échantillon biologique du sujet et surveiller la formation de complexes de liaison formés par la liaison des peptides tels que définis dans la revendication 1 par des anticorps dans l'échantillon qui sont spécifiques pour les antigènes bactériens.


### SEQUENCE LISTING

<110> Szymanski, Christine
   Nothaft, Harald
<120> PEPTIDE CONTAINING MULTIPLE N-LINKED GLYCOSYLATION SEQUONS
   <130> PPCT21583
   <150> US 61/379,896
   <151> 2010-09-03
   <160> 31
   <170> PatentIn version 3.5
<210> 1
   <211> 7
   <212> PRT
   <213> Campylobacter jejuni
<400> 1
   Lys Ile Asp Leu Asn Asn Thr
   1               5
<210> 2
   <211> 264
   <212> DNA
   <213> Campylobacter jejuni
<400> 2
   ctcgagttca taaaaaattt caagcaacat gaaaaaatta agatagatct taataataca
   60
   aagatagatc ttaataatac aaagatagat cttaataata caaagataga tcttaataat
   120
   acaaagatag atcttaataa tacaaagata gatcttaata atacaaagat agatcttaat
   180
   aatacaaaga tagatcttaa taatacaaag atagatctta ataatacaaa gatagaatta
   240
   tcgcaattaa aaaaagagct cgag
   264
<210> 3
   <211> 27
   <212> DNA
   <213> Artificial Sequence
   <220>
   <223> primer
<400> 3
   atggccatgg ataataaaaa atcagcc
   27
<210> 4
   <211> 35
   <212> DNA
   <213> Artificial Sequence
   <220>
   <223> primer
<400> 4
   tttctcgagt ttgcttcctt tggcatcatt tatcg
   35
<210> 5
   <211> 28
   <212> DNA
   <213> Artificial Sequence
   <220>
   <223> primer
<400> 5
   aaactcgagt tcataaaaaa tttcaagc
   28
<210> 6
   <211> 27
   <212> DNA
   <213> Artificial Sequence
   <220>
   <223> primer
<400> 6
   atatctcgag ctcttttttt aattgcg
   27
<210> 7
   <211> 468
   <212> PRT
   <213> Camplyobacter fetus fetus/Campylobacter jejuni
<400> 7
   Met Lys Tyr Leu Leu Pro Thr Ala Ala Ala Gly Leu Leu Leu Leu Ala
   1               5                   10                  15
   Ala Gln Pro Ala Met Ala Met Asp Asn Lys Lys Ser Ala Ala Gln Gln
               20                  25                  30
   Gln Ile Pro Pro Met Pro Val Thr Val Met Gln Ala Lys Met Gly Asp
           35                  40                  45
   Ile Pro Ile Val Leu Ser Phe Asn Gly Gln Thr Val Ser Asp Met Asp
       50                  55                  60
   Val Val Leu Lys Ala Lys Val Ala Gly Thr Ile Glu Lys Gln Phe Phe
   65                  70                  75                  80
   Lys Ala Gly Ala Ser Val Lys Glu Gly Asp Lys Leu Tyr Gln Ile Asp
                   85                  90                  95
   Glu Ala Lys Tyr Arg Ala Ala Tyr Asp Ser Ala Phe Ala Asn Leu Gln
               100                 105                 110
   Val Ser Gln Ala Asn Leu Lys Asn Ala Glu Ser Asp Phe Asp Arg Ala
           115                 120                 125
   Lys Lys Leu Gln Glu Lys Ser Ala Ile Ser Gln Lys Glu Tyr Asp Ala
       130                 135                 140
   Ala Leu Ala Ala Tyr Glu Ser Ala Lys Ala Ser Val Leu Ala Ser Lys
   145                 150                 155                 160
   Ala Asn Val Gln Thr Ala Lys Ile Asp Leu Asp Tyr Ser Thr Val Thr
                   165                 170                 175
   Ala Pro Phe Ser Gly Val Leu Gly Asp Thr Leu Lys Asp Val Gly Ser
               180                 185                 190
   Tyr Val Ser Ser Ser Asp Ala Asp Leu Val Arg Leu Thr Lys Leu Asn
           195                 200                 205
   Pro Ile Phe Val Lys Phe Gly Ile Ser Asp Val Asp Lys Leu Asn Ile
       210                 215                 220
   Ala Gln Lys Thr Arg Thr Lys Glu Trp Glu Gln Leu Asn Ser Leu Val
   225                 230                 235                 240
   Thr Leu Ser Met Gln Asn Gly Asp Tyr Asn Gly Thr Leu Thr Phe Ile
                   245                 250                 255
   Asp Asn Val Ile Asp Glu Gly Ser Gly Thr Val Asn Ala Lys Ala Met
               260                 265                 270
   Phe Glu Asn Asn Ser Thr Gln Ile Arg Pro Gly Ile Phe Ala Ser Val
           275                 280                 285
   Asn Val Tyr Gly Phe Tyr Gln Lys Asn Gly Phe Lys Ile Pro Gln Val
       290                 295                 300
   Ala Ile Leu Gln Asp Leu Ala Ser Pro Phe Val Tyr Thr Leu Lys Asp
   305                 310                 315                 320
   Gly Lys Val Ala Lys Asn Pro Ile Lys Ile Val Ser Gln Asp Ser Thr
                   325                 330                 335
   Ser Ala Val Ile Ser Ser Gly Ile Asn Asp Gly Asp Leu Ile Ile Met
               340                 345                 350
   Asp Asn Phe Lys Lys Ile Asn Ile Gly Gln Pro Val Gln Ala Ile Asn
           355                 360                 365
   Asp Ala Lys Gly Ser Lys Leu Glu Phe Ile Lys Asn Phe Lys Gln His
       370                 375                 380
   Glu Lys Ile Lys Ile Asp Leu Asn Asn Thr Lys Ile Asp Leu Asn Asn
   385                 390                 395                 400
   Thr Lys Ile Asp Leu Asn Asn Thr Lys Ile Asp Leu Asn Asn Thr Lys
                   405                 410                 415
   Ile Asp Leu Asn Asn Thr Lys Ile Asp Leu Asn Asn Thr Lys Ile Asp
               420                 425                 430
   Leu Asn Asn Thr Lys Ile Asp Leu Asn Asn Thr Lys Ile Asp Leu Asn
           435                 440                 445
   Asn Thr Lys Ile Glu Leu Ser Gln Leu Lys Lys Glu Leu Glu His His
       450                 455                 460
   His His His His
   465
<210> 8
   <211> 469
   <212> PRT
   <213> Camplyobacter fetus fetus/Campylobacter jejuni
<400> 8
   Met Lys Tyr Leu Leu Pro Thr Ala Ala Ala Gly Leu Leu Leu Leu Ala
   1               5                   10                  15
   Ala Gln Pro Ala Met Ala Met Val Ile Ile Lys Asn Phe Lys Gln His
               20                  25                  30
   Glu Lys Ile Lys Ile Asp Leu Asn Asn Thr Lys Ile Asp Leu Asn Asn
           35                  40                  45
   Thr Lys Ile Asp Leu Asn Asn Thr Lys Ile Asp Leu Asn Asn Thr Lys
       50                  55                  60
   Ile Asp Leu Asn Asn Thr Lys Ile Asp Leu Asn Asn Thr Lys Ile Asp
   65                  70                  75                  80
   Leu Asn Asn Thr Lys Ile Asp Leu Asn Asn Thr Lys Ile Asp Leu Asn
                   85                  90                  95
   Asn Thr Lys Ile Glu Leu Ser Gln Leu Lys Lys Glu Pro Met Asp Asn
               100                 105                 110
   Lys Lys Ser Ala Ala Gln Gln Gln Ile Pro Pro Met Pro Val Thr Val
           115                 120                 125
   Met Gln Ala Lys Met Gly Asp Ile Pro Ile Val Leu Ser Phe Asn Gly
       130                 135                 140
   Gln Thr Val Ser Asp Met Asp Val Val Leu Lys Ala Lys Val Ala Gly
   145                 150                 155                 160
   Thr Ile Glu Lys Gln Phe Phe Lys Ala Gly Ala Ser Val Lys Glu Gly
                   165                 170                 175
   Asp Lys Leu Tyr Gln Ile Asp Glu Ala Lys Tyr Arg Ala Ala Tyr Asp
               180                 185                 190
   Ser Ala Phe Ala Asn Leu Gln Val Ser Gln Ala Asn Leu Lys Asn Ala
           195                 200                 205
   Glu Ser Asp Phe Asp Arg Ala Lys Lys Leu Gln Glu Lys Ser Ala Ile
       210                 215                 220
   Ser Gln Lys Glu Tyr Asp Ala Ala Leu Ala Ala Tyr Glu Ser Ala Lys
   225                 230                 235                 240
   Ala Ser Val Leu Ala Ser Lys Ala Asn Val Gln Thr Ala Lys Ile Asp
                   245                 250                 255
   Leu Asp Tyr Ser Thr Val Thr Ala Pro Phe Ser Gly Val Leu Gly Asp
               260                 265                 270
   Thr Leu Lys Asp Val Gly Ser Tyr Val Ser Ser Ser Asp Ala Asp Leu
           275                 280                 285
   Val Arg Leu Thr Lys Leu Asn Pro Ile Phe Val Lys Phe Gly Ile Ser
       290                 295                 300
   Asp Val Asp Lys Leu Asn Ile Ala Gln Lys Thr Arg Thr Lys Glu Trp
   305                 310                 315                 320
   Glu Gln Leu Asn Ser Leu Val Thr Leu Ser Met Gln Asn Gly Asp Tyr
                   325                 330                 335
   Asn Gly Thr Leu Thr Phe Ile Asp Asn Val Ile Asp Glu Gly Ser Gly
               340                 345                 350
   Thr Val Asn Ala Lys Ala Met Phe Glu Asn Asn Ser Thr Gln Ile Arg
           355                 360                 365
   Pro Gly Ile Phe Ala Ser Val Asn Val Tyr Gly Phe Tyr Gln Lys Asn
       370                 375                 380
   Gly Phe Lys Ile Pro Gln Val Ala Ile Leu Gln Asp Leu Ala Ser Pro
   385                 390                 395                 400
   Phe Val Tyr Thr Leu Lys Asp Gly Lys Val Ala Lys Asn Pro Ile Lys
                   405                 410                 415
   Ile Val Ser Gln Asp Ser Thr Ser Ala Val Ile Ser Ser Gly Ile Asn
               420                 425                 430
   Asp Gly Asp Leu Ile Ile Met Asp Asn Phe Lys Lys Ile Asn Ile Gly
           435                 440                 445
   Gln Pro Val Gln Ala Ile Asn Asp Ala Lys Gly Ser Lys Leu Glu His
       450                 455                 460
   His His His His His
   465
<210> 9
   <211> 467
   <212> PRT
   <213> Camplyobacter fetus fetus/Campylobacter jejuni
<400> 9
   Met Lys Tyr Leu Leu Pro Thr Ala Ala Ala Gly Leu Leu Leu Leu Ala
   1               5                   10                  15
   Ala Gln Pro Ala Met Ala Met Asp Asn Lys Lys Ser Ala Ala Gln Gln
               20                  25                  30
   Gln Ile Pro Pro Met Pro Val Thr Val Met Gln Ala Lys Met Gly Asp
           35                  40                  45
   Ile Pro Ile Val Leu Ser Phe Asn Gly Thr Val Ser Asp Met Asp Val
       50                  55                  60
   Val Leu Lys Ala Lys Val Ala Gly Thr Ile Glu Lys Gln Phe Phe Lys
   65                  70                  75                  80
   Ala Gly Ala Ser Val Lys Glu Gly Asp Lys Leu Tyr Gln Ile Asp Glu
                   85                  90                  95
   Ala Lys Tyr Arg Ala Ala Tyr Asp Ser Ala Phe Ala Asn Leu Gln Val
               100                 105                 110
   Ser Gln Ala Asn Leu Lys Asn Ala Glu Ser Asp Phe Asp Arg Ala Lys
           115                 120                 125
   Lys Leu Gln Glu Lys Ser Ala Ile Ser Gln Lys Glu Tyr Asp Ala Ala
       130                 135                 140
   Leu Ala Ala Tyr Glu Ser Ala Lys Ala Ser Phe Ile Lys Asn Phe Lys
   145                 150                 155                 160
   Gln His Glu Lys Ile Lys Ile Asp Leu Asn Asn Thr Lys Ile Asp Leu
                   165                 170                 175
   Asn Asn Thr Lys Ile Asp Leu Asn Asn Thr Lys Ile Asp Leu Asn Asn
               180                 185                 190
   Thr Lys Ile Asp Leu Asn Asn Thr Lys Ile Asp Leu Asn Asn Thr Lys
           195                 200                 205
   Ile Asp Leu Asn Asn Thr Lys Ile Asp Leu Asn Asn Thr Lys Ile Asp
       210                 215                 220
   Leu Asn Asn Thr Lys Ile Glu Leu Ser Gln Leu Lys Lys Glu Ala Ser
   225                 230                 235                 240
   Val Leu Ala Ser Lys Ala Asn Val Gln Thr Ala Lys Ile Asp Leu Asp
                   245                 250                 255
   Tyr Ser Thr Val Thr Ala Pro Phe Ser Gly Val Leu Gly Asp Thr Leu
               260                 265                 270
   Lys Asp Val Gly Ser Tyr Val Ser Ser Ser Asp Ala Asp Leu Val Arg
           275                 280                 285
   Leu Thr Lys Leu Asn Pro Ile Phe Val Lys Phe Gly Ile Ser Asp Val
       290                 295                 300
   Asp Lys Leu Asn Ile Ala Gln Lys Thr Arg Thr Lys Glu Trp Glu Gln
   305                 310                 315                 320
   Leu Asn Ser Leu Val Thr Leu Ser Met Gln Asn Gly Asp Tyr Asn Gly
                   325                 330                 335
   Thr Leu Thr Phe Ile Asp Asn Val Ile Asp Glu Gly Ser Gly Thr Val
               340                 345                 350
   Asn Ala Lys Ala Met Phe Glu Asn Asn Ser Thr Gln Ile Arg Pro Gly
           355                 360                 365
   Ile Phe Ala Ser Val Asn Val Tyr Gly Phe Tyr Gln Lys Asn Gly Phe
       370                 375                 380
   Lys Ile Pro Gln Val Ala Ile Leu Gln Asp Leu Ala Ser Pro Phe Val
   385                 390                 395                 400
   Tyr Thr Leu Lys Asp Gly Lys Val Ala Lys Asn Pro Ile Lys Ile Val
                   405                 410                 415
   Ser Gln Asp Ser Thr Ser Ala Val Ile Ser Ser Gly Ile Asn Asp Gly
               420                 425                 430
   Asp Leu Ile Ile Met Asp Asn Phe Lys Lys Ile Asn Ile Gly Gln Pro
           435                 440                 445
   Val Gln Ala Ile Asn Asp Ala Lys Gly Ser Lys Leu Glu His His His
       450                 455                 460
   His His His
   465
<210> 10
   <211> 63
   <212> PRT
   <213> Campylobacter jejuni
<400> 10
   Lys Ile Asp Leu Asn Asn Thr Lys Ile Asp Leu Asn Asn Thr Lys Ile
   1               5                   10                  15
   Asp Leu Asn Asn Thr Lys Ile Asp Leu Asn Asn Thr Lys Ile Asp Leu
               20                  25                  30
   Asn Asn Thr Lys Ile Asp Leu Asn Asn Thr Lys Ile Asp Leu Asn Asn
           35                  40                  45
   Thr Lys Ile Asp Leu Asn Asn Thr Lys Ile Asp Leu Asn Asn Thr
       50                  55                  60
<210> 11
   <211> 33
   <212> DNA
   <213> Artificial Sequence
   <220>
   <223> primer
<400> 11
   aatatatacc atgggttgca ctcgggcaat tcg
   33
<210> 12
   <211> 35
   <212> DNA
   <213> Artificial Sequence
   <220>
   <223> primer
<400> 12
   atatatctcg agcgttaccc tgcggatgtt ttcgg
   35
<210> 13
   <211> 49
   <212> DNA
   <213> Artificial Sequence
   <220>
   <223> primer
<400> 13
   tatatggcca tggcaacacc aattccattt tcttattcta aaaatctgg
   49
<210> 14
   <211> 49
   <212> DNA
   <213> Artificial Sequence
   <220>
   <223> primer
<400> 14
   ttaaactcga gatcatttgt ccatccttca tctgtaggta caaagtacc
   49
<210> 15
   <211> 36
   <212> DNA
   <213> Artificial Sequence
   <220>
   <223> primer
<400> 15
   atatatatcc atggctgctg atgatgttgt tgattc
   36
<210> 16
   <211> 37
   <212> DNA
   <213> Artificial Sequence
   <220>
   <223> primer
<400> 16
   atatactcga gtcgcctgac acgatttcct gcacagg
   37
<210> 17
   <211> 44
   <212> DNA
   <213> Artificial Sequence
   <220>
   <223> primer
<400> 17
   gcaattaaaa aaagagatcg agcaccacca ccaccaccac tgag
   44
<210> 18
   <211> 44
   <212> DNA
   <213> Artificial Sequence
   <220>
   <223> primer
<400> 18
   ctcagtggtg gtggtggtgg tgctcgatct ctttttttaa ttgc
   44
<210> 19
   <211> 45
   <212> DNA
   <213> Artificial Sequence
   <220>
   <223> primer
<400> 19
   gctgcccagc cggcgatggc gatggttatc ataaaaaatt tcaag
   45
<210> 20
   <211> 45
   <212> DNA
   <213> Artificial Sequence
   <220>
   <223> primer
<400> 20
   cttgaaattt tttatgataa ccatcgccat cgccggctgg gcagc
   45
<210> 21
   <211> 46
   <212> DNA
   <213> Artificial Sequence
   <220>
   <223> primer
<400> 21
   atatctcgag gcggccgcaa gctgaggaga ctgtgaccat gactcc
   46
<210> 22
   <211> 34
   <212> DNA
   <213> Artificial Sequence
   <220>
   <223> primer
<400> 22
   aattctgcag taattttgtt taactttaag aagg
   34
<210> 23
   <211> 38
   <212> DNA
   <213> Artificial Sequence
   <220>
   <223> primer
<400> 23
   atatatatcc atgggaggtg gcggatcaga agtgaagc
   38
<210> 24
   <211> 31
   <212> DNA
   <213> Artificial Sequence
   <220>
   <223> primer
<400> 24
   atatggtacc ttagcagccg gatctcagtg g
   31
<210> 25
   <211> 28
   <212> DNA
   <213> Artificial Sequence
   <220>
   <223> primer
<400> 25
   aaaataaata taggacaacc ggttcagg
   28
<210> 26
   <211> 29
   <212> DNA
   <213> Artificial Sequence
   <220>
   <223> primer
<400> 26
   tatctcgagt cactcttttt ttaattgcg
   29
<210> 27
   <211> 506
   <212> PRT
   <213> Campylobacter jejuni/Mus musculus
   <400> 27
   Met Lys Ile Lys Thr Gly Ala Arg Ile Leu Ala Leu Ser Ala Leu Thr
   1               5                   10                  15
   Thr Met Met Phe Ser Ala Ser Ala Leu Ala Lys Ile Glu Glu Gly Lys
               20                  25                  30
   Leu Val Ile Trp Ile Asn Gly Asp Lys Gly Tyr Asn Gly Leu Ala Glu
           35                  40                  45
   Val Gly Lys Lys Phe Glu Lys Asp Thr Gly Ile Lys Val Thr Val Glu
       50                  55                  60
   His Pro Asp Lys Leu Glu Glu Lys Phe Pro Gln Val Ala Ala Thr Gly
   65                  70                  75                  80
   Asp Gly Pro Asp Ile Ile Phe Trp Ala His Asp Arg Phe Gly Gly Tyr
                   85                  90                  95
   Ala Gln Ser Gly Leu Leu Ala Glu Ile Thr Pro Asp Lys Ala Phe Gln
               100                 105                 110
   Asp Lys Leu Tyr Pro Phe Thr Trp Asp Ala Val Arg Tyr Asn Gly Lys
           115                 120                 125
   Leu Ile Ala Tyr Pro Ile Ala Val Glu Ala Leu Ser Leu Ile Tyr Asn
       130                 135                 140
   Lys Asp Leu Leu Pro Asn Pro Pro Lys Thr Trp Glu Glu Ile Pro Ala
   145                 150                 155                 160
   Leu Asp Lys Glu Leu Lys Ala Lys Gly Lys Ser Ala Leu Met Phe Asn
                   165                 170                 175
   Leu Gln Glu Pro Tyr Phe Thr Trp Pro Leu Ile Ala Ala Asp Gly Gly
               180                 185                 190
   Tyr Ala Phe Lys Tyr Glu Asn Gly Lys Tyr Asp Ile Lys Asp Val Gly
           195                 200                 205
   Val Asp Asn Ala Gly Ala Lys Ala Gly Leu Thr Phe Leu Val Asp Leu
       210                 215                 220
   Ile Lys Asn Lys His Met Asn Ala Asp Thr Asp Tyr Ser Ile Ala Glu
   225                 230                 235                 240
   Ala Ala Phe Asn Lys Gly Glu Thr Ala Met Thr Ile Asn Gly Pro Trp
                   245                 250                 255
   Ala Trp Ser Asn Ile Asp Thr Ser Lys Val Asn Tyr Gly Val Thr Val
                   260                 265                 270
   Leu Pro Thr Phe Lys Gly Gln Pro Ser Lys Pro Phe Val Gly Val Leu
               275                 280                 285
   Ser Ala Gly Ile Asn Ala Ala Ser Pro Asn Lys Glu Leu Ala Lys Glu
       290                 295                 300
   Phe Leu Glu Asn Tyr Leu Leu Thr Asp Glu Gly Leu Glu Ala Val Asn
   305                 310                 315                 320
   Lys Asp Lys Pro Leu Gly Ala Val Ala Leu Lys Ser Tyr Glu Glu Glu
                   325                 330                 335
   Leu Ala Lys Asp Pro Arg Ile Ala Ala Thr Met Glu Asn Ala Gln Lys
               340                 345                 350
   Gly Glu Ile Met Pro Asn Ile Pro Gln Met Ser Ala Phe Trp Tyr Ala
           355                 360                 365
   Val Arg Thr Ala Val Ile Asn Ala Ala Ser Gly Arg Gln Thr Val Asp
       370                 375                 380
   Glu Ala Leu Lys Asp Ala Gln Thr Asn Ser Ser Ser Asn Asn Asn Asn
   385                 390                 395                 400
   Asn Asn Asn Asn Asn Asn Leu Gly Ile Glu Gly Arg Ile Ser Glu Phe
                   405                 410                 415
   Gly Ser Ser Arg Val Glu Phe Ile Lys Asn Phe Lys Gln His Glu Lys
               420                 425                 430
   Ile Lys Ile Asp Leu Asn Asn Thr Lys Ile Asp Leu Asn Asn Thr Lys
           435                 440                 445
   Ile Asp Leu Asn Asn Thr Lys Ile Asp Leu Asn Asn Thr Lys Ile Asp
       450                 455                 460
   Leu Asn Asn Thr Lys Ile Asp Leu Asn Asn Thr Lys Ile Asp Leu Asn
   465                 470                 475                 480
   Asn Thr Lys Ile Asp Leu Asn Asn Thr Lys Ile Asp Leu Asn Asn Thr
                   485                 490                 495
   Lys Ile Glu Leu Ser Gln Leu Lys Lys Glu
               500                 505
<210> 28
   <211> 28
   <212> DNA
   <213> Artificial Sequence
   <220>
   <223> primer
<400> 28
   aaaataaata taggacaacc ggttcagg
   28
<210> 29
   <211> 34
   <212> DNA
   <213> Artificial Sequence
   <220>
   <223> primer
<400> 29
   aatttaagct ttgttagcag ccggatctca gtgg
   34
<210> 30
   <211> 514
   <212> PRT
   <213> Campylobacter jejuni/Mus musculus
<400> 30
   Met Lys Ile Lys Thr Gly Ala Arg Ile Leu Ala Leu Ser Ala Leu Thr
   1               5                   10                  15
   Thr Met Met Phe Ser Ala Ser Ala Leu Ala Lys Ile Glu Glu Gly Lys
               20                  25                  30
   Leu Val Ile Trp Ile Asn Gly Asp Lys Gly Tyr Asn Gly Leu Ala Glu
           35                  40                  45
   Val Gly Lys Lys Phe Glu Lys Asp Thr Gly Ile Lys Val Thr Val Glu
       50                  55                  60
   His Pro Asp Lys Leu Glu Glu Lys Phe Pro Gln Val Ala Ala Thr Gly
   65                  70                  75                  80
   Asp Gly Pro Asp Ile Ile Phe Trp Ala His Asp Arg Phe Gly Gly Tyr
                   85                  90                  95
   Ala Gln Ser Gly Leu Leu Ala Glu Ile Thr Pro Asp Lys Ala Phe Gln
               100                 105                 110
   Asp Lys Leu Tyr Pro Phe Thr Trp Asp Ala Val Arg Tyr Asn Gly Lys
           115                 120                 125
   Leu Ile Ala Tyr Pro Ile Ala Val Glu Ala Leu Ser Leu Ile Tyr Asn
       130                 135                 140
   Lys Asp Leu Leu Pro Asn Pro Pro Lys Thr Trp Glu Glu Ile Pro Ala
   145                 150                 155                 160
   Leu Asp Lys Glu Leu Lys Ala Lys Gly Lys Ser Ala Leu Met Phe Asn
                   165                 170                 175
   Leu Gln Glu Pro Tyr Phe Thr Trp Pro Leu Ile Ala Ala Asp Gly Gly
               180                 185                 190
   Tyr Ala Phe Lys Tyr Glu Asn Gly Lys Tyr Asp Ile Lys Asp Val Gly
           195                 200                 205
   Val Asp Asn Ala Gly Ala Lys Ala Gly Leu Thr Phe Leu Val Asp Leu
       210                 215                 220
   Ile Lys Asn Lys His Met Asn Ala Asp Thr Asp Tyr Ser Ile Ala Glu
   225                 230                 235                 240
   Ala Ala Phe Asn Lys Gly Glu Thr Ala Met Thr Ile Asn Gly Pro Trp
                   245                 250                 255
   Ala Trp Ser Asn Ile Asp Thr Ser Lys Val Asn Tyr Gly Val Thr Val
               260                 265                 270
   Leu Pro Thr Phe Lys Gly Gln Pro Ser Lys Pro Phe Val Gly Val Leu
           275                 280                 285
   Ser Ala Gly Ile Asn Ala Ala Ser Pro Asn Lys Glu Leu Ala Lys Glu
       290                 295                 300
   Phe Leu Glu Asn Tyr Leu Leu Thr Asp Glu Gly Leu Glu Ala Val Asn
   305                 310                 315                 320
   Lys Asp Lys Pro Leu Gly Ala Val Ala Leu Lys Ser Tyr Glu Glu Glu
                   325                 330                 335
   Leu Ala Lys Asp Pro Arg Ile Ala Ala Thr Met Glu Asn Ala Gln Lys
               340                 345                 350
   Gly Glu Ile Met Pro Asn Ile Pro Gln Met Ser Ala Phe Trp Tyr Ala
           355                 360                 365
   Val Arg Thr Ala Val Ile Asn Ala Ala Ser Gly Arg Gln Thr Val Asp
       370                 375                 380
   Glu Ala Leu Lys Asp Ala Gln Thr Asn Ser Ser Ser Asn Asn Asn Asn
   385                 390                 395                 400
   Asn Asn Asn Asn Asn Asn Leu Gly Ile Glu Gly Arg Ile Ser Glu Phe
                   405                 410                 415
   Gly Ser Ser Arg Val Glu Phe Ile Lys Asn Phe Lys Gln His Glu Lys
               420                 425                 430
   Ile Lys Ile Asp Leu Asn Asn Thr Lys Ile Asp Leu Asn Asn Thr Lys
           435                 440                 445
   Ile Asp Leu Asn Asn Thr Lys Ile Asp Leu Asn Asn Thr Lys Ile Asp
       450                 455                 460
   Leu Asn Asn Thr Lys Ile Asp Leu Asn Asn Thr Lys Ile Asp Leu Asn
   465                 470                 475                 480
   Asn Thr Lys Ile Asp Leu Asn Asn Thr Lys Ile Asp Leu Asn Asn Thr
                   485                 490                 495
   Lys Ile Glu Leu Ser Gln Leu Lys Lys Glu Ile Glu His His His His
               500                 505                 510
   His His
<210> 31
   <211> 150
   <212> PRT
   <213> Campylobacter jejuni
<400> 31
   Met Gln Arg Phe Lys Lys Trp Phe Leu Ser Ile Ile Lys Asn Phe Lys
   1               5                   10                  15
   Gln His Glu Lys Ile Lys Ile Asp Leu Asn Asn Thr Lys Ile Asp Leu
               20                  25                  30
   Asn Asn Thr Lys Ile Asp Leu Asn Asn Thr Lys Ile Asp Leu Asn Asn
           35                  40                  45
   Thr Lys Ile Asp Leu Asn Asn Thr Lys Ile Asp Leu Asn Asn Thr Lys
       50                  55                  60
   Ile Asp Leu Asn Asn Thr Lys Ile Asp Leu Asn Asn Thr Lys Ile Asp
   65                  70                  75                  80
   Leu Asn Asn Thr Lys Ile Glu Leu Ser Gln Leu Lys Lys Glu His Tyr
                   85                  90                  95
   Lys Val Leu Asp Phe His Leu Arg Lys Ile Thr Pro Gln Ala Phe Leu
               100                 105                 110
   Glu Ile Val Glu Ile His Leu Ala Glu Ser Cys Asn Leu Asn Cys Phe
           115                 120                 125
   Gly Cys Asn His Phe Ser Gln Ile Ala Glu Lys Glu Phe Pro Asp Ile
       130                 135                 140
   Glu Ile Phe Lys Lys Asp
   145                 150